(19) Europäisches Patentamt
European Patent Office
Office européen des brevets

(11) Publication number: 0 540 742 A1

(12) EUROPEAN PATENT APPLICATION
published in accordance with Art. 158(3) EPC

(21) Application number: 91913310.8

(51) Int. Cl.$^5$: C07H 21/04

(22) Date of filing: 26.07.91

(86) International application number:
PCT/JP91/01007

(87) International publication number:
WO 92/01704 (06.02.92 92/04)

(30) Priority: 26.07.90 JP 198123/90

(43) Date of publication of application:
12.05.93 Bulletin 93/19

(84) Designated Contracting States:
CH DE ES FR GB IT LI NL

(71) Applicant: Shudo, Koichi, Prof. Dr.
6-102, 25 Higashiyama 2-chome
Meguro-ku Tokyo 153(JP)

(72) Inventor: SHUDO, Koichi
25-6-102, Higashiyama 2-chome
Meguro-ku, Tokyo 153(JP)
Inventor: HASHIMOTO, Yuichi
16-18, Nakaochiai 3-chome
Shinjuku-ku, Tokyo 161(JP)
Inventor: FUJIMORI, Shizuyoshi
664-7, Marubayashi, Nogimachi
Shimotsuga-gun, Tochigi-ken 329-01(JP)

(74) Representative: Werner, Hans-Karsten, Dr. et al
Deichmannhaus am Hauptbahnhof
W-5000 Köln 1 (DE)

(54) **OLIGODEOXYRIBONUCLEOTIDES.**

(57) An oligodeoxynucleotide including an oligonucleotide comprising 5'-phosphates of a nucleoside, formed by combining 2-deoxy-L-*erythro*-pentose with a nucleic acid base in the form of a β-anomer, linked with each other through 3'→5' phosphodiester linkages; and another oligodeoxynucleotide including an oligonucleotide comprising 5'-phosphates of a nucleoside, formed by combining 2-deoxy-D-ribose with a nucleic acid base in the form of a β-anomer, and 5'-phosphates of another nucleoside, formed by combining 2-deoxy-L-*erythro*-pentose with a nucleic acid base in the form of a β-anomer, linked with each other alternatingly through 3'→5' phosphodiester linkages. Each of the oligodeoxynucleotides combines specifically with a natural oligonucleotide having a complementary base sequence and is useful as an antisense DNA having an activity of inhibiting gene expression. These oligodeoxynucleotides can be efficiently produced by the solid phase method using a phosphoramidite derivative containing a 2-deoxy-β-L-*erythro*-pentofuranosyl moiety.

EP 0 540 742 A1

## Technical Field

The present invention relates to an oligodeoxyribonucleotide.

More specifically, the present invention relates to a polynucleotides formed by successively combining an enantiomeric isomer of a natural deoxyribonucleotide, and to a polynucleotide formed by alternately combining a natural deoxyribonucleotide and an enantiomeric isomer of a natural deoxyribonucleotide. Each of the oligodeoxyribonucleotides is an antisense nucleotide which binds to a natural nucleic acid having a complementary base sequence.

## Background Art

Certain oligodeoxyribonucleotides and their analogues are known to regulate gene expression. They regulate gene expression by inhibiting a process such as DNA replication or transcription, the splicing of mRNA or translation to a protein by complementary binding to deoxyribonucleic acid (DNA) or to messenger RNA (mRNA), which is a sort of ribonucleic acid (RNA).

These nucleotides are particularly referred to as antisense nucleotides and are capable of being hybridized with a specific deoxyribonucleic acid or ribonucleic acid, or of exhibiting a certain cellular-biological activity upon being introduced into a cell. Consequently, they are expected to be useful as anti-viral agents and chemotherapeutical agents.

Some natural oligodeoxyribonucleotides as well as unnatural oligodeoxyribonucleotides such as a nucleotide having a nucleoside bond in alpha-configuration or a nucleotide having a modified phosphoric acid ester linkage are known as such antisense nucleotides (see, as reviews, P.S. Miller et al., Oligonucleotides inhibitors of gene expression in living cells: New opportunities in drug design, Ann. Rep. Med. Chem., 23, 295-304, 1988; and E. Uhlmann et al., Antisense Oligonucleotides, A new therapeutic principle, Chem. Rev., 90, 543, 1990).

Dimeric enantiomers of ribonucleotide having L-erythro-pentose have been reported as nucleotide derivatives (Tazawa et al., Biochemistry, 9, 3499, 1970). The synthesis of L-$(dAdT)_3$ was reported as an oligomer of deoxyribonucleotide having 2-deoxy-L-erythro-pentose as a sugar moiety in beta-configuration (C.P. Rakoczy et al. Chem. Abs. 110, 115261t, 1988). However, the presence of interaction between these oligomers and DNA or RNA has not been reported. The oligomers disclosed have a self-complementary sequence which results in self-dimerization, and consequently they are unsuitable for the purpose of interacting them with natural DNA or RNA.

Furthermore, the presence of interaction between L-oligodeoxyribonucleotide comprising 2-deoxy-L-erythro-pentose as a sugar moiety in beta-configuration and DNA or RNA has not been reported. It was reported that the 18-mer of L-deoxyuridine comprising uridine as a base-containing component of RNA has no interaction with DNA having a complementary sequence (D.J. Anderson et al., Nucleosides and ncleotides, 3, 499, 1984).

Accordingly, an object of the present invention is to provide oligonucleotides which comprise L-deoxyribonucleotides as component nucleotides and specifically bind to DNA or RNA having a complementary sequence to that of said oligonucleotide.

The inventors of the present invention conducted an intensive study aimed at achieving the foregoing object, and as a result, they found that an oligonucleotide (an enantiomeric oligomer of a natural oligodeoxyribonucleotide), which is formed by the 3'→ 5' phosphodiester linkage between L-nucleotides, i.e., the 5'-phosphoric acid esters of a nucleoside (an enantiomeric nucleoside of a natural nucleoside: L-nucleoside) that comprises 2-deoxy-L-erythro-pentose and a nucleic acid base linked together in beta-configuration, specifically binds to a natural nucleic acid such as DNA or RNA having a complementary sequence to that of the oligonucleotide, and thus achieved the present invention.

The inventors of the present invention also found that an oligonucleotide which is formed by alternately combining the above-described L-nucleotide with D-nucleotide, i.e., the 5'-phosphoric acid ester of a natural nucleoside (D-nucleoside) that comprises 2-deoxy-D-ribose and a nucleic acid base linked together in beta-configuration by the 3'→ 5' phosphodiester linkage, strongly binds to natural DNA and RNA having a complementary sequence to that of said oligonucleotide, and thus achieved the present invention.

## Disclosure of the Invention

The present invention provides an oligodeoxynucleotide comprising an oligonucleotide formed by linking the 5'-phosphoric acid ester of a nucleoside that comprises 2-deoxy-L-erythro-pentose and a

nucleic acid base linked together in beta-configuration by the 3'→ 5' phosphodiester linkage, and the above-defined oligodeoxyribonucleotide which specifically binds to a natural oligonucleotide comprising a complementary sequence to that of said oligonucleotide.

The present invention also provides an oligodeoxynucleotide comprising an oligonucleotide formed by alternately linking the 5'-phosphoric acid ester of a nucleoside that comprises 2-deoxy-D-ribose and a nucleic acid base linked together in beta-configuration with the 5'-phosphoric acid ester of a nucleoside that comprises 2-deoxy-L-erythro-pentose and a nucleic acid base linked together in beta-configuration by the 3' → 5' phosphodiester linkage, and the above-defined oligodeoxyribonucleotide which specifically binds to a natural oligonucleotide comprising a complementary sequence to that of said oligonucleotide.

The present invention further provide a phosphoramidite derivative useful for the preparation of the above-described oligodeoxyribonucleotide by solid phase synthesis.

Brief Description of Drawings

Fig. 1 shows the mixing curve of $(L-dA)_6$ of the present invention with poly U and poly dT.

Fig. 2 shows the mixing curve of natural type $(D-dA)_6$ as a reference with poly U and poly dT.

Fig. 3 shows the mixing curve of $(L-dA)_{12}$ of the present invention with poly U and poly dT.

Fig. 4 shows the mixing curve of $(LD-dA)_{12}$ of the present invention with poly U and poly dT.

In Figs. 1 through 4, ● represents the results for poly U and □ represents the results for poly dT.

Fig. 5 shows the melting curve of $(L-dA)_6$ of the present invention with poly U and poly dT.

Fig. 6 shows the melting curve of natural type $(D-dA)_6$ as a reference with poly U and poly dT.

Fig. 7 shows the melting curve of $(LD-dA)_{12}$ and $(L-dA)_{12}$ of the present invention and natural type $(D-dA)_{12}$ as a reference with poly U.

Fig. 8 shows the melting curve of $(LD-dA)_{12}$ and $(L-dA)_{12}$ of the present invention and natural type $(D-dA)_{12}$ as a reference with poly dT.

Fig. 9 shows hydrolysis of $(L-dA)_6$ of the present invention and natural type $(D-dA)_6$ as a reference by phosphodiesterase.

Fig. 10 shows hydrolysis of $(LD-dA)_{12}$ of the present invention and natural type $(D-dA)_{12}$ as a reference by phosphodiesterase.

Best Mode for Carrying Out the Invention

The oligodeoxyribonucleotide of the present invention comprises an oligonucleotide formed by linking the 5'-phosphoric acid ester (L-nucleotide) of a nucleoside (L-nucleoside) that comprises 2-deoxy-L-erythro-pentose and a nucleic acid base linked together in beta-configuration by the 3'→ 5' phosphodiester linkage.

2-Deoxy-L-erythro-pentose represented by the formula set out below is a component element of the L-nucleoside and is the enantiometric isomer of 2-deoxy-D-ribose, which is a component element of a nucleoside (D-nucleoside) contained in natural DNA.

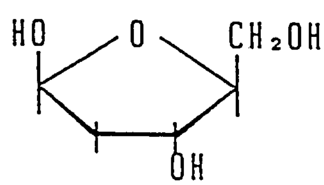

This compound is known to the art and can be prepared by the method of R. E. Deriatz et al., J. Chem. Soc., 1949, page 1879.

The L-nucleoside composing oligodeoxyribonucleotide of the present invention is the nucleoside comprising the above-described 2-deoxy-L-erythro-pentose as sugar moiety and a nucleic acid base linked together in beta-configuration. The nucleic acid base may be a nucleic acid base such as adenine, guanine, cytosine, thymine, urasil, or modified bases thereof.

Examples of the L-nucleoside include, for example, L-deoxyadenosine, L-deoxyguanosine, L-deoxycytidine, L-deoxyuridine, L-thymidine, L-4-acetyldeoxycytidine, L-5-(carboxyhydroxymethyl) deoxyuridine, L-5-carboxymethylaminomethyl-2-thio-deoxyuridine, L-5-carbox-

ymethylaminomethyldeoxyuridine, L-N6-isopentenyldeoxyadenosine, L-1-methyldeoxyadenosine, L-1-methyldeoxypseudouridine, L-1-methyldeoxyguanosine, L-2,2-dimethyldeoxyguanosine, L-2-methyldeoxyadenosine, L-2-methyldeoxyguanosine, L-3-methyldeoxycytidine, L-5-methyldeoxycytidine, L-N6-methyldeoxyadenosine, and L-7-methyldeoxyguanosine.

Among these L-nucleosides, L-deoxyadenosine [L-dA: 9-(2-deoxy-beta-L-erythro-pentofuranosil)-9H-purine-6-amine]; L-deoxyguanosine [L-dG: 2-amino-9-(2-deoxy-beta-L-erythro-pentofuranosil)-9H-purine-6(1H)-one]; L-deoxycytidine [L-dC: 4-amino-1-(2-deoxy-beta-L-erythro-pentofuranosil)-2(1H)-pyrimidinone]; and L-deoxythymidine [L-dT: 1-(2-deoxy-beta-L-erythro-pentofuranosil)-5-methyl-2,4(1H,3H)-pyrimidinedione] are preferable.

These L-nucleosides can be prepared using the above-described 2-deoxy-L-erythro-pentose and a nucleic acid base such as adenine according to a known method which comprises linking 2-deoxy-D-erythro-pentoseto a nucleic acid base such as adenine by beta-glycoside bond. Examples of these methods include the method of M. J. Robins (J. Org. Chem., 35, 636, 1970) and the method of A. Holly (Coll. Czech. Chem. Commun., 37, 4072, 1972). The L-nucleotides, the components of the L-oligodeoxyribonucleotide of the present invention, are the 3',5'-phosphoric acid esters of these L-nucleosides.

The oligodeoxyribonucleotide of the present invention is the compound represented by the following formula (I).

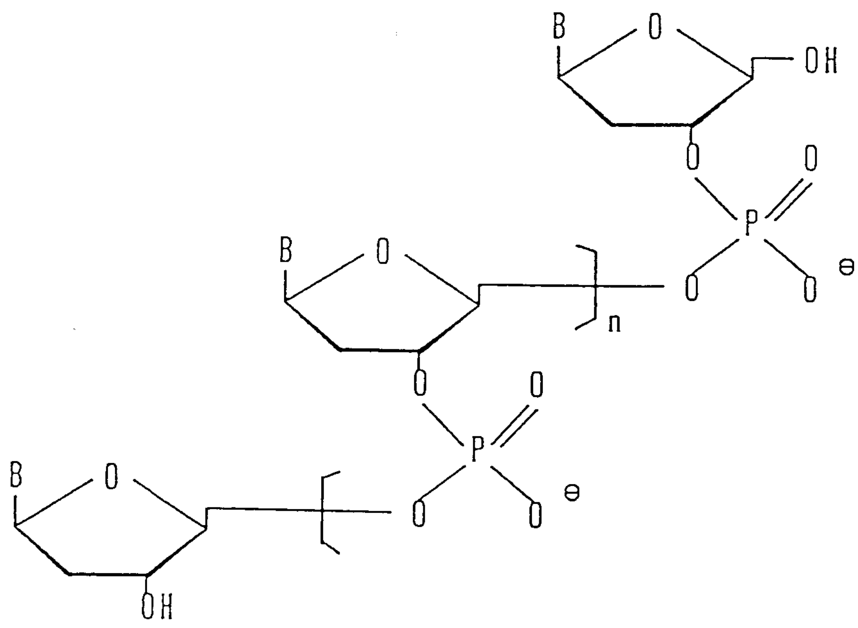

(I)

Another oligodeoxyribonucleotide of the present invention is the compound represented by the following formula (II).

LD-oligomer

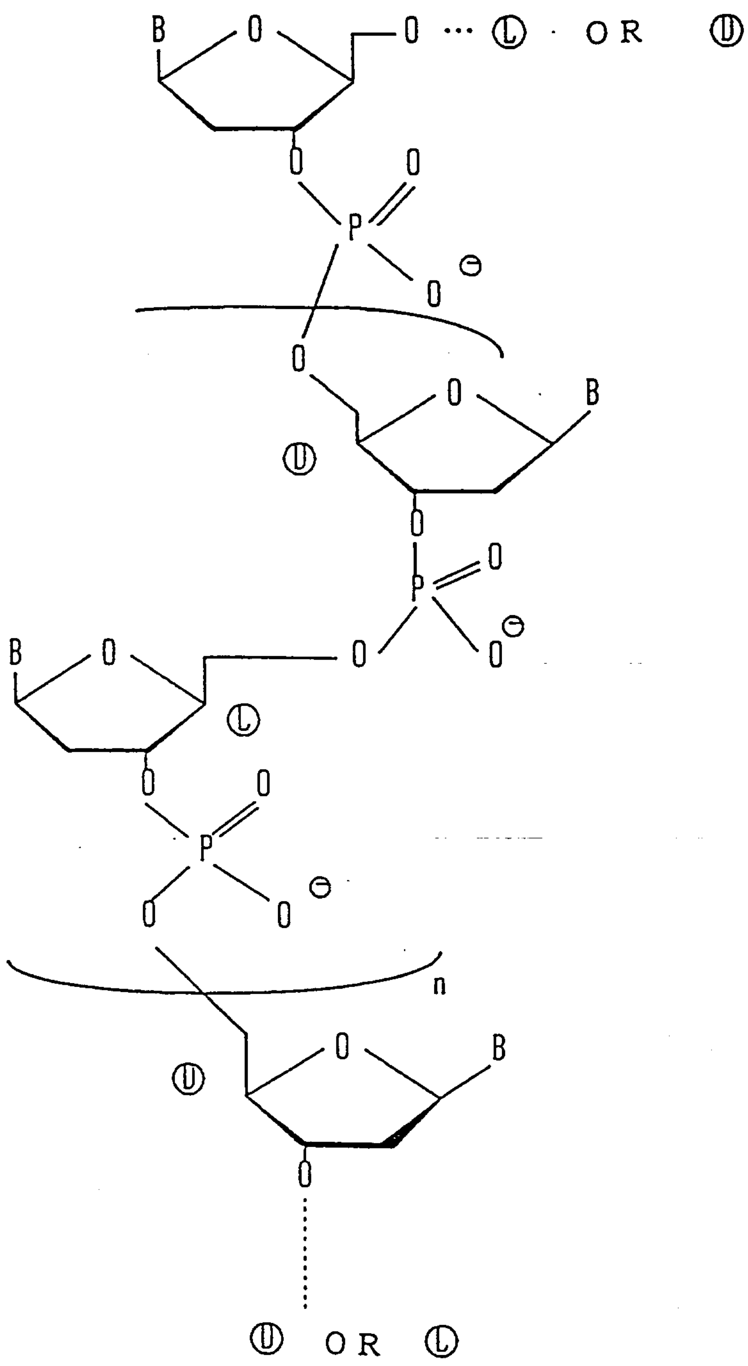

(II)

In the formulas (I) and (II), n represents an integer of from 2 to 60, preferably from 2 to 40, most preferably from 4 to 20. B represents a nucleic acid base which may have a protective group. The nucleic acid bases may be entirely the same bases or may be different bases. The salt of these compounds also fall within the scope of the invention. Examples of such salts include base-addition salts such as triethylammonium salt or sodium salt.

The oligodeoxyribonucleotides of the present invention can be prepared by a block synthesis utilizing a liquid phase procedure using the triphosphate ester derivative of the above-described L-nucleoside, or alternatively, by a solid phase synthesis. An example of the method for preparation will be set out below with reference to the oligodeoxyribonucleotide compound represented by the above-described formula (I) wherein n is 5 and each B represents adenine [abbreviated as $(L-dA)_6$].

2-Deoxy-L-erythro-pentose and adenine are applied to glycosilation reaction according to the method of Robins (J. Org. Chem., 35, 636, 1970) or stereoselective glycosilation method (Z. Kazimierczuk et al., 106, 6379, 1984), and after the resulting L-dA is protected, for example, as N,N-dibenzoyl-5'-dimethoxytrityl derivative, triesterification is carried out according to phosphoric acid esterification using 8-quinoyldihydogenphosphite, which is then followed by dimerization reaction. The tetramer which is obtained by successive tetramerization after the dimerization is further reacted with the dimer to obtain hexamer having protective groups. The protective groups are then removed by an ordinary method and the desired $(L-dA)_6$ can be obtained by purifying the resulting product by, for example, liquid chromatography. As the protective group, acetyl, benzoyl, or N,N-dimethylmethylene (for NH group); monomethoxytrityl or trityloxyacetyl (for 5'-OH group); and beta-cyanoethyl or o-chlorophenyl (for phosphoryl group) may be used besides those described above. The cleavage of the protective group may be conducted according to the method of H. Takaku (Chem. Lett., 1983, 1661) or the like. The triethylammonium salt of $(L-dA)_6$ can be obtained using triethylammonium acetate in the liquid chromatography procedure.

The above process will be shown as scheme 1. In the scheme, the meaning of the symbols is as follows: Bz: benzoyl; DMTr: dimethoxytrityl; TMSCl: chlorotrimethylsilyl; BzCl: benzoylchloride; DMTrCl: Dimethoxytrityl chloride; QS-: 8-quinolinesulfonylchloride; Q: 8-quinolyl; $QO-PO(OH)_2$: 8-quinolinedihydrogenphosphate; CeOH: 2-cyanoethanol; $Ac_2O$: acetic anhydride; Ac: acetyl; Te: 1H-tetrazol; and QSNT: 8-quinolinesulfonyl-3-nitro-1,2,4-triazole.

Scheme 1

L-deoxyadenosine

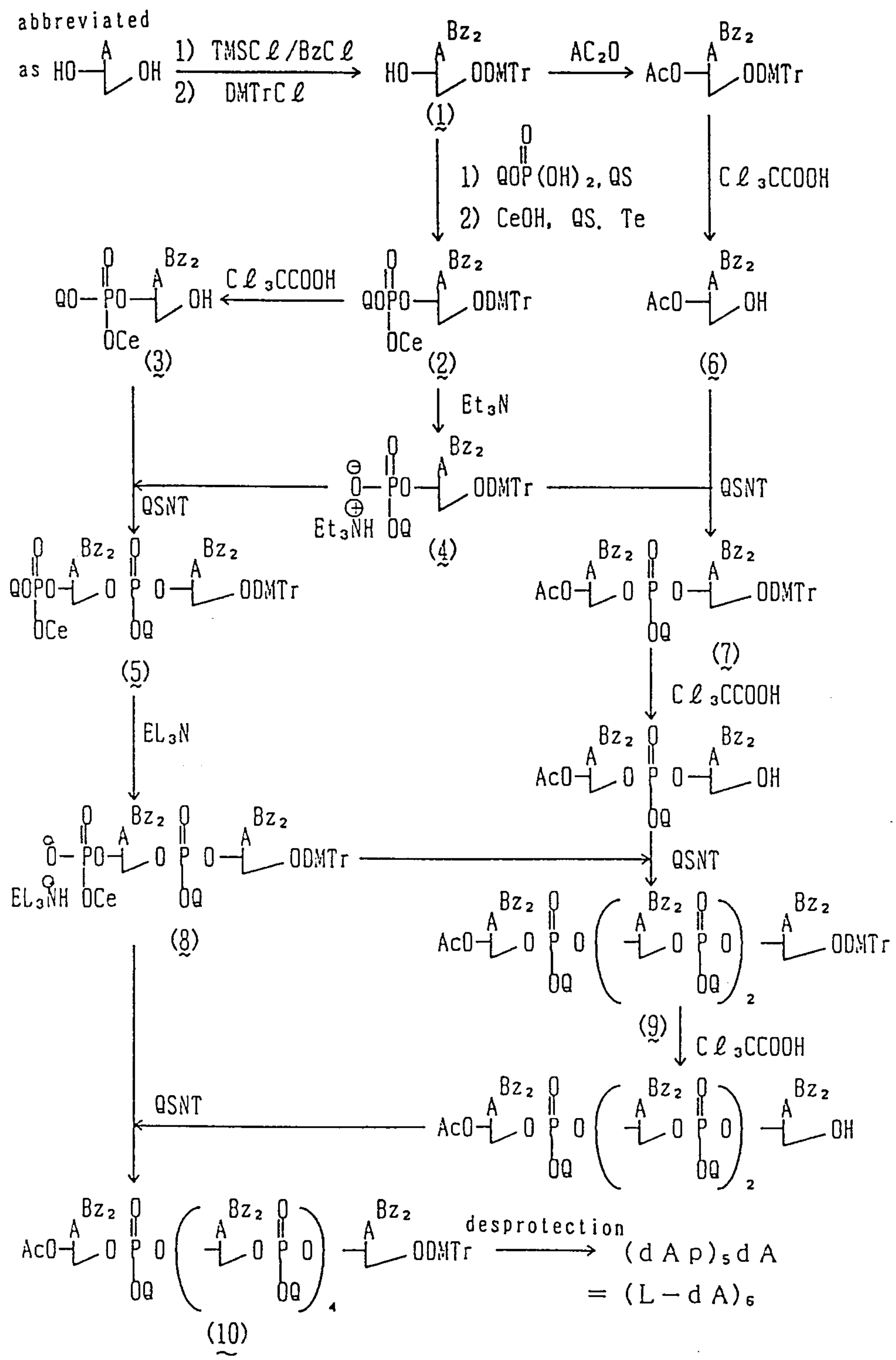


In the solid phase synthesis, the preparation can be conducted by utilizing a commercially available DNA synthesizer using a phosphoramidite derivative of the present invention which is a protected L-nucleoside described above.

The phosphoramidite of the present invention is the compound represented by the formula set out below.

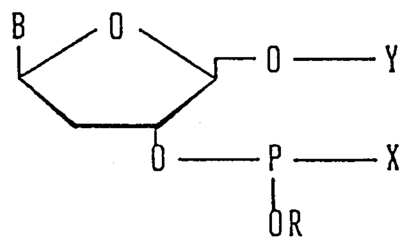

In the formula, B represents a nucleic acid base which may have a protective group. Examples of the nucleic acid bases include, for example, adenine, guanine, cytosine, thymine, urasil, and deoxycytosine. The protective groups of the nucleic acid base include, for example, acetyl, benzoyl, N,N－dimethyl－methylene, and isobutyryl. Y represents a protective group for hydroxyl group which may be, for example, dimethoxytrityl group, monomethoxytrityl, trityloxyacetyl or the like. R represents cyanoethyl group or a lower alkyl group, and X represents an amino group or a protected amino group. Examples of the protected amino group include, for example, diisopropylamino group and morpholine.

The phosphoramidite derivative of the present invention can be prepared from the L－nucleoside described above according to, for example, the method reported by N. D. Sinha et al. (Nucleic Acids Res., 12, 4539, 1984).

Examples of the preferred phosphoramidite include, for example,

3'－O－cyanoethyl－N,N－diisopropylphosphoramidite derivative of 6－benzoylamino－9－[2－deoxy－5－(4,4'－dimethoxytrityl)－beta－L－erythro－pentofuranosyl]－9H－purine;

3'－O－cyanoethyl－N,N－diisopropylphosphoramidite derivative of 1－[2－deoxy－5－(4,4'－dimethoxytrityl)－beta－L－erythro－pentofuranosyl]－5－methyl－2,4(1H,3H)－pyrimidinedione;

3'－O－cyanoethyl－N,N－diisopropylphosphoramidite derivative of 6－benzoylamino－1－[2－deoxy－5－(4,4'－dimethoxytrityl)－beta－L－erythro－pentofuranosyl]－2(1H)－pyrimidinone;

3'－O－cyanoethyl－N,N－diisopropylphosphoramidite derivative of 2－isobutyrylamino－9－[2－deoxy－5－(4,4'－dimethoxytrityl)－beta－L－erythro－pentofuranosyl]－1,9－dihydro－6H－purine－6－one;

3'－O－cyanoethyl－N,N－diisopropylphosphoramidite derivative of 6－isobutyrylamino－1－[2－deoxy－5－(4,4'－dimethoxytrityl)－beta－L－erythro－pentofuranosyl]－2(1H)－pyrimidinone;

3'－O－cyanoethyl－N,N－diisopropylphosphoramidite derivative of 6－(N,N－dimethylamidino)－9－[2－deoxy－5－(4,4'－dimethoxytrityl)－beta－L－erythro－pentofuranosyl]－9H－purine; and

3'－O－cyanoethyl－N,N－diisopropylphosphoramidite derivative of 2－(N,N－dimethylamidino)－9－[2－deoxy－5－(4,4'－dimethoxytrityl)－beta－L－erythro－pentofuranosyl]－1,9－dihydro－6H－purine－6－one.

An example of the process for preparing the phosphoramidite derivative of the present invention will be described based on the scheme 2 set out below.

Scheme 2

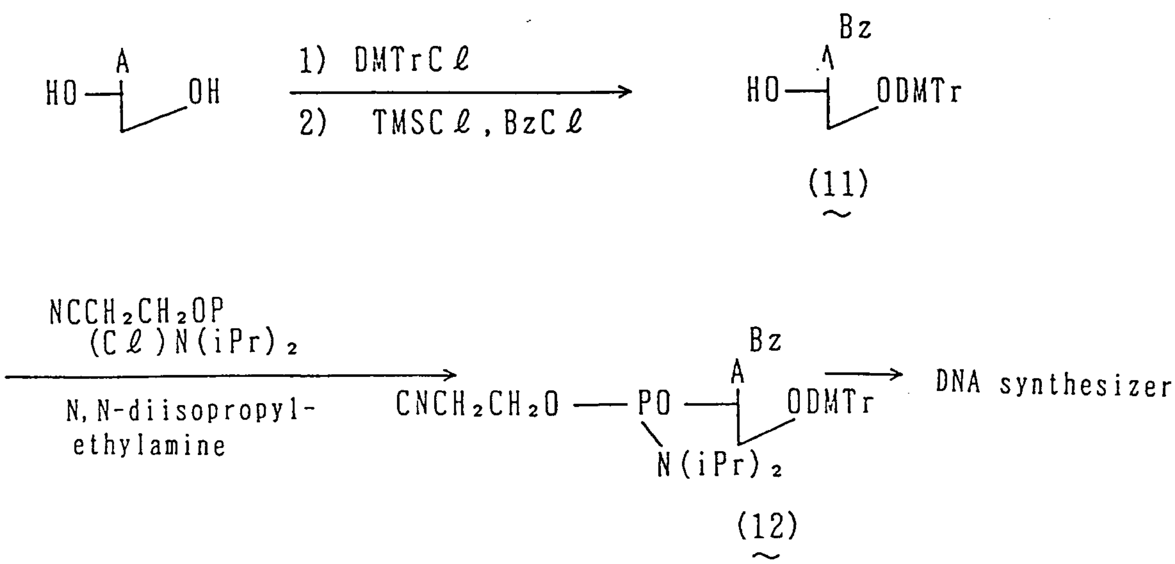


(11)

(12)

The oligodeoxyribonucleotide of the present invention can be prepared by a solid phase process using a commercially available DNA synthesizer (from, for example, Applied Biosystem), which comprises polynucleotide chain extension by reacting nucleoside or oligonucleotide immobilized on a solid phase with the above−described phosphoramidite derivative of N−benzoyl−L−deoxyadenosine used as a reagent.

The process comprises, for example, (a) nucleotide bond forming process comprising a first step of forming nucleotide bond by reacting the above−described L−phosphoramidite derivative with the terminal nucleoside of a deoxyribonucleoside immmobilized to a solid phase or an oligodeoxyribonucleotide immobilized to a solid phase; a second step of acylating 5'−hydroxyl group, and a third step of oxidizing the phosphorus; (b) nucleotide extension process comprising a step of conducting from the first step to the third step of the above−described process (a) using the above−described L−phosphoramidite after the 5'−protective group of immobilized nucleotide obtained in the above process (a) is removed; (c) process of repeating the above process (b), if desired; and (d) process of removing protective groups comprising the steps of removing the protective groups for the phosphoramidite moiety of the immobilized protected oligodeoxyribonucleotide obtained above and carrying out the cleavage from the solid phase; removing the protective groups for the base moiety of the oligodeoxyribonucleotide; and removing the 5'−protective group.

The process will be hereinafter explained as an example with reference to the process comprising the use of the phosphoramidite derivative wherein R is cyanoethyl group and Y is dimethoxytrityl group by using a commercially available CPG (controlled pore glass) column to which 5'−tritylated nucleoside having an appropriately protected terminal is immobilized. The oligodeoxyribonucleotide of the present invention can be prepared by the steps of removing 5'−trityl group of the nucleotide immobilized to the CPG column using the solution of trichloroacetic acid in dichloromethane; forming the nucleotide bond by reacting the solution of tetrazole in acetonitrile with the solution containing a protected phosphoramidite such as L−phosphoramidite in acetonitrile; acetylating the unreacted 5'−hydroxyl group by the solution of 1−methylimidazole and acetic anhydride in tetrahydrofuran; forming nucleotide bond by oxidizing phosphorus by iodine in tetrahydrofuran containing pyridine and water; further extending nucleotide by repeating the above steps using appropriate L−phosphoramidite derivatives; succesively removing cyanoethyl group by the treatment of aqueous ammonia at room temperature after the desired nucleotide is obtained; removing the protective groups for the base moiety of the protected oligodeoxyribonucleotide by the treatment of aqueous ammonia at an elevated temperature, and removing 5'−protective group using acetic acid.

In addition, the process for preparing the oligodeoxyribonucleotide of the present invention formed by alternately binding a natural nucleoside and L−nucleoside comprises, for example, (a) nucleotide bond forming process comprising a first step of forming nucleotide bond by reacting the above−described L−phosphoramidite derivative with the terminal nucleoside of a deoxyribonucleoside immmobilized to a solid phase or an oligodeoxyribonucleotide immobilized to a solid phase; a second step of acylating 5'−hydroxyl group, and a third step of oxidizing the phosphorus; (b) nucleotide extension process comprising a step of conducting from the first step to the third step of the above−described process (a) using a natural type D−phosphoramidite derivative after the 5'−protective group of immobilized nucleotide obtained in the above process (a) is removed, followed by conducting from the first step to the third step of the above−described process (a) using the L−phosphoramidite after the 5'−protective group of the resulting immobilized nucleotide is removed; (c) process of repeating the above process (b), if desired; and (d) process of removing protective groups comprising the steps of removing the protective groups for the phosphoramidite moiety of the resulting immobilized protected oligodeoxyribonucleotide and carrying out the cleavage from the solid phase; removing the protective groups for the base moiety of the oligodeoxyribonucleotide; and removing the 5'−protective group.

In a process as described above, the oligodeoxyribonucleotide of the present invention having a natural type nucleoside or a natural type oligonucleoside bound to the terminal of the oligomer of L−nuclotide can be prepared by using a solid phase to which a natural type nucleoside or a natural type oligonucleotide is immobilized. The oligodeoxyribonucleotide of the present invention can also be prepared efficiently by using a solid phase to which L−nucleotide of L−oligonucleotide is immobilized. Although the nucleoside or oligonucleoside immobilized to a solid phase can serve per se as a linker, such nucleotide or oligonucleotide may optionally be immobilized to a solid phase through other linkers well known to one of ordinary skill in the art. Such linkers may, for example, be trityloxyalkylalcohol, trityloxyalkylamine or the like which are readily removable from the CPG column. These linkers can be removed from a solid phase or the oligodeoxyribonucleotide of the present invention by a method well known to one of ordinary skill in the art.

The oligodeoxyribonucleotides having a natural nucleoside at the terminal of the oligomer of L−nucleotide are also included in the oligodeoxyribonucleotide of the present invention. For example, where

D−dA−(L−dA)$_6$ having a terminal natural nucleoside is prepared according to the method described above, (L−dA)$_6$ i.e., the hexaorligomer of L−nucleotide, can be obtained by hydrolysing the diester bond of the terminal natural adenylic acid using phosphodiesterase. For the hydrolysis, the condition described by L. Tazawa (Biochemistry, 9, 3499, 1970) can be applied. A phosphodiesterase such as bovine spleen phosphodiesterase or snake venom phosphodiesterase may suitably be used. By the treatment with bovine spleen phosphodiesterase, an L−oligodeoxyribonucleotide having a remaining phosphoric acid ester at the 3'−terminal can be obtained. On the other hand, by the treatment with snake venom phosphodiesterase, natural nucleotides at the 3'− and 5'−terminal are hydrolyzed to afford the L−oligodeoxyribonucleotide having no natural nucleotide either end.

According to the process described above, the oligodeoxyribonucleotide comprising L−nucleosides such as L−dA, L−dG, L−dC, or L−dT binding to each other in an arbitrary sequence (hereinafter referred to L−oligodeoxyribonucleotide) can be prepared. In the same manner, the oligodeoxyribonucleotide comprising an arbitrary sequence formed by alternately binding a natural deoxynucleoside and L−dA, L−dG, L−dC, L−dT or the like by the 3' → 5' phosphodiester linkage (hereinafter referred to as LD−oligodeoxyribonucleotide) can be prepared. A natural deoxynucleotide contained in the LD−oligodeoxyribonucleotide may be, for example, D−dA, D−dG, D−dC, D−dT or the like, or natural D−nucleosides corresponding to the L−nucleosides described above. Either a natural nucleoside or the L−nucleoside may be present at the 3'−end, and either a natural nucleoside or the L−nucleoside may be present at the 5'−end. In consequence, both the oligonucleotides having L−nucleosides at both ends and the oligonucleotide having natural nucleosides at both ends are included among the LD−oligodeoxyribonucleotides.

The oligodeoxyribonucleotide of the present invention comprises the above−described L−oligodeoxyribonucleotide or LD−oligodeoxyribonucleotide, which may further comprise a natural−type ribonucleoside or a natural type oligoribonucleotide which is bound to the 3'− or 5'−end or at both ends of the L−oligodeoxyribonucleotide or the LD−oligodeoxyribonucleotide.

The oligodeoxyribonucleotide of the present invention includes, for example, the nucleotides having the following base sequences:
GTAGAGGATACCGA (HTLV−III virus promoter sequence);
GGTGTGTTTACCCT (EB virus promoter sequence);
AATACTCATACTCTTC (Amp $^r$ promoter sequence);
CTTCTCATACTCATAA (Amp $^r$ promoter sequence);
GAAAGGCGTCGACGGAG (Prn II site);
TTTCGTCATTC (HTLV−III splicing site); and
AAAGTCTGGG (HTLV−III splicing site).
However, the oligodeoxyribonucleotide of the present invention is not limited to these examples.

The oligodeoxyribonucleotide of the present invention comprises L−oligodeoxyribonucleotide or LD−oligodeoxyribonucleotide and binds to a nucleic acid comprising a natural type oligonucleotide (a natural type nucleic acid sequence) that comprises a complementary sequence to its nucleotide sequence. As a result, the oligodeoxyribonucleotide of the present invention binds to a natural nucleic acid and acts as an antisense DNA that inhibits occurence of inherent biochemical reaction, in particular, gene expression. For example, where the component nucloside of the L−oligodeoxyribonucleotide contained in the oligodeoxyribonucleotide of the present invention is L−dA, the component ribonucleoside of the complementary RNA is natural uridine, and the component deoxyribonucleoside is natural thymidine. For another example, the natural RNA oligomer UGAC and the natural DNA oligomer TGAC are complementary nucleotide to L−ACTG, which is an L−oligodeoxyribonucleotide composed of L−dA, L−dC, L−dT, and L−dG. The oligonucleotide of the present invention having a longer sequence will become able to bind to a natural DNA. For example, (L−dA)$_6$ binds to natural RNA having the complementary sequence (poly U), while it does not bind to natural DNA having the complementary sequence (poly dT). On the other hand, (L−dA)$_{12}$ binds to both poly U and poly dT and it does not bind to poly G, poly C, poly A, poly dG, poly dC, or poly dA.

Although no particular theory is purported, the binding between the oligodeoxyribonucleotide of the present invention and the complementary nucleid acid is presumed to be formed by chemical bonds such as hydrogen bond or hydrophobic bond. The presence of the binding force can be detected by a physicochemical method apparent to one of ordinary skill in the art, i.e, a known method for detecting a binding of nucleic acids (interaction). Examples of the method include the measurement of the change of the intensity of ultraviolet absorption after both nucleotides are mixed, the measurement of the melting point (Tm) of the mixture of L−oligodeoxyribonucleotide and the complementary nucleic acid, the measurement of the change in chemical shifts using NMR, and the measurement of circular dichroism.

Where the change in the intensity of ultraviolet absorption is measured after a natural nucleic acid is mixed with oligodeoxyribonucleotide of the present invention comprising L-oligodeoxyribonucleotide or LD-oligodeoxyribonucleotide, the binding between the two nucleotides can be demonstrated by, for example, heating the mixture containing $(L-dA)_6$ or $(L-dA)_{12}$ as a nucleotide of the present invention and poly uridine (poly U) as the complementary RNA to the nucleotide at about 80 °C for approximately 10 minutes and successively cooling the mixture for several hours at 0 °C, and then measuring the degree of absorption at an ultraviolet region to obtain a mixing curve (such as, for example, shown in Fig. 1A) showing the interaction between the two nucleotides. In general, the hypochromicity of the ultraviolet absorption is approximately 10 to 30% when the binding of the oligodeoxyribonucleotide of the present invention to a complementary nucleic acid is measured by the method described above.

Among the oligodeoxyribonucleotides of the present invention, the ribonucleotide containing L-oligodeoxyribonucleotide interacts with the RNA which is complementary to the sequence of the L-oligodeoxyribonucleotide, and it does not bind to non-complementary RNA. On the other hand, this oligodeoxyribonucleotide weakly interacts with a natural D-oligodeoxyribonucleotide (DNA), and in consequence, the interaction of this oligodeoxyribonucleotide with a natural nucleic acid is obviously specific to a complementary RNA. No interaction or only very weak interaction can be detected by the method described above between this oligodeoxyribonucleotide and a non-complementary RNA or DNA.

Among the oligodeoxyribonucleotides of the present invention, the ribonucleotide containing LD-oligodeoxyribonucleotide strongly binds to both RNA and DNA having the complementary sequence to the LD-oligodeoxyribonucleotide sequence.

The oligodeoxyribonucleotide of the present invention acts as an antisense DNA which complementarily binds to a specific DNA or RNA and inhibits processes such as DNA replication or transcription, mRNA splicing or translation to a protein. Consequently, the oligodeoxyribonucleotide of the present invention can be used as a regulator of gene expression for the treatment of various diseases or diagnoses, and is useful as, for example, an anti-viral agent.

The present invention will be further illustrated hereinafter by way of examples. However, the present invention is not limited to these examples. In examples, the compound number corresponds to the compound number in the scheme.

## Examples

### Example 1: The preparation of $(L-dA)_6$ by a liquid phase method

This compound is prepared according to the reported method using D-deoxyadenosine (Z. Kazimierczuk et al.) and the method for preparing an oligomer (H. Takaku et al.).

L-Deoxyadenosine, 7.5 g (30 mmol), m.p. 188-191 °C, $[\alpha]_D^{25}$ +26.5 ° (c=1.05, $H_2O$), was treated with chlorotrimethylsilane and the amino groups were successively dibenzoylated with benzoylchloride, and the result was then treated with dimethoxytritylchloride to afford 9.75 g of N, 5'-protected compound (1). Compound (1) was converted to phosphoric acid ester at the 3-position using 8-quinolylhydrogenphosphate in the presence of quinolinesulfonyl chloride, and then the addition of cyanoethanol was conducted to afford 8.11 g of protected phosphorylated monomer (2). Condensation was carried out using 1.80 g of des-tritylated compound (3) obtained by treating compound (2) with trichloroacetic acid and 3.88 g of des-cyanoethylated compound (4) obtained by treating with triethylamine to give 2.89 g of dimer (5). Compound (1) 1.15 g was separately acetylated at the 3'-position using acetic anhydride and then destritylated using trichloroacetic acid to afford 0.41 g of 3'-acetyl protected monomer (6), and then compound (6) and 1.30 g of compound (4) were condensed to give 0.88 g of 3'-acetyl protected dimer (7). Des-tritylated compound 0.63 g obtained from compound (7) was condensed with 1.43 g of descyanoethylated compound (8) obtained from (5) to give 0.48 g of tetramer (9). Des-tritylated compound obtained from (9) and 0.466 g of compound (8) were condensed to afford 253 mg of protected hexamer (10). Des-protection was carried out by successive treatment with pyridinealdoxime-tetramethylguanidine salt, concentrated aqueous ammonia, and 80% acetic acid, and final purification was conducted by using HPLC (ODS reverse-phase preparative column, 0.1M-triethylamine acetate-9% acetonitrile), and 43.6 g of triethylamine salt of the desired compound was obtained after desaltation and lyophilization (total yield 0.5%).

NMR (400MHz, $D_2O$, TMS) δ ppm: 5.70 (3H,m,1'-H), 5.83 (1H,m,1'-H), 5.98 (1H,m,1'-H), 6.17 (1H,t,1'-H).

The NMR spectrum and the chromatographic retention time of this compound are completely identical with those of $(D-dA)_6$ prepared from D-deoxyadenosine by the same method, and they have the same

CD spectra shape but are completely opposite in sign.

Example 2: 3'-O-cyanoethyl-N,N-diisopropylphosphoramidite of 6-benzoylamino-9-[2-deoxy-5-(4,4'-dimethoxytrityl)-beta-L-erythro-pentofuranosyl]-9H-purine

According to the method for preparing the protected D-deoxyadenosine (G.S.Ti et al., J. Am. Chem. Soc., 104, 1316, 1982), 2.5 g (10 mmol) of L-deoxyadenosine was protected at the 5'-position using 4,4'-dimethoxytrityl chloride in pyridine in the presence of triethylamine, and N-benzoyl compound (11) 4.39 g (66.7%) was obtained by successive treatment with trimethylchlorosilane and benzoyl chloride. Compound (11) 329 mg (0.5 mmol) was dissolved in dry tetrahydrofuran in an argon atmosphere and then 0.35 ml (2 mM) of diisopropylethylamine and 0.223 ml (1 mmol, Aldrich) of beta-cyanoethylmonochloro-phosphoramidite were added and stirring was continued for 35 minutes at room temperature. The mixture was concentrated to dryness after the salts precipitated were separated by filtration. The residue was dissolved in argon-saturated ethyl acetate, which was then washed with chilled saturated sodium chloride solution and 10% sodium hydrogen carbonate solution and dried. After the solvent was evaporated, the residue was subjected to silica gel column chromatography and eluted by using methylene chloride: ethyl acetate: triethylamine (45:45:10). After the fractions were separately concentrated and dried by azeotropy using benzene, the residue was dissolved in a small volume of ethyl acetate and treated with n-hexane to afford 247 mg of compound (12) as a colorless amorphous product (57.5%, phosphorous diastereomers).
NMR (400MHz, $CDCl_3$, TMS) $\delta$ ppm: 1.19 (12H,m,isopropyl $CH_3$), 3.77 (6H,d,methoxy $CH_3$), 6.51 (1H,m,1'-H), 9.04 (1H,s,NH).

This compound had an NMR spectrum completely the same as that of the corresponding phosphoramidite derived from D-deoxyadenosine.

Example 3: 3'-O-cyanoethyl-N,N-diisopropylphosphoramidite of 1-[2-deoxy-5-(4,4'-dimethoxytrityl)-beta-L-erythro-pentofuranosyl]-5-methyl-2,4(1H,3H)-pyrimidinedione

According to the method of M. Hoffer (Chem. Ber., 93, 2777, 1960), methyl 2-deoxy-3,5-bis-O-(4-methylbenzoyl)-L-erhythro-pentofuranoside was obtaind from 5 g (37.3 mmol) of 2-deoxy-L-ribose, which was then chlorinated without purification to afford 10.0 g (69.0%) of 1-chloro-2-deoxy-3,5-bis-O-(4-methylbenzoyl)-alpha-L-erythro-pentofuranose (m.p. 113-114 °C).

A mixture of 12.6 g (0.1 mol) of thymine, 26 ml (0.125 mol) of hexamethyldisilazane and 1 ml of trimethylsilyl chloride was refluxed for 3 hours and then 18.9 g of colorless oil was obtained by distillation under a reduced pressure after excess disilazane was evaporated off (74-76 °C/0.65 mmHg, 70.0%). Crystalline 5-methyl-2,4-bis-(trimethylsilyloxy)pyrimidine was obtained by letting the oil stand as it was (m.p. 73-74 °C).

1-Chloro-2-deoxy-3.5-bis-O-(4-methylbenzoyl)-alpha-L-erythro-pentofuranose 17.7 g (46 mmol) was added to the mixture of 15.7 g (58.1 mmol) of 5-methyl-2,4-bis-(trimethylsilyloxy)-pyrimidine, 2.55 g (18.3 mmol) of para-nitrophenol, and 175 ml of dry dichloromethane and stirring was continued overnight at room temperature (for 20 hours). A small volume of ethanol was added to the reaction mixture to decompose excess silyl compound and insoluble precipitate was removed by filtration using celite. A small amount of ethanol was added to the residue obtained by concentrating the filtrate to afford crystals, which were collected by filtration and washed with a small amount of ethanol. The crystals were dried to give 22.9 g of white powder. Recrystallization was carried out using 1.5 liter of ethanol to give 1-[3,5-bis-O-(4-methylbenzoyl)-2-deoxy-beta-L-erythro-pentofuranosyl]-5-methyl-2,4-(1H,3H)-pyrimidinedione as colorless needles (m.p. 199-201 °C).
NMR (400MHz, $CDCl_3$, TMS) $\delta$ ppm: 6.47 (1H,dd,J=5.5, 9.2Hz,1'H)

| Anal. (for $C_{26}H_{26}N_2O_7$) | | | |
|---|---|---|---|
| Calcd. | C: 65.26%; | H: 5.48%; | N: 5.86% |
| Found | C: 65.27%; | H: 5.51%; | N: 5.78% |

$[\alpha]_D^{25}$ + 71.9 ° (c=0.528, chloroform)

Dry methanol (300 ml) was saturated with gaseous ammonia under ice cooling and then 16.7 g (34.9 mmol) of the above-obtained compound was added and stirring was continued for 6 hours. The mixture was again saturated with gaseous ammonia and stirred overnight (20 hours). Concentration was carried out after the complete consumption of the starting material was observed by thin layer chromatography, and the

residue was dissolved in water and washed once with chloroform and twice with ether after insoluble materials were removed by filtration. Water was removed by evaporation under a reduced pressure, and toluene was added to dry the residue by azeotropic distillation. The residue was washed with a small volume of ethanol to afford 7.31 g (86.6%) of 1-(2-deoxy-beta-L-erythro-pentofuranosyl)-5-methyl-2,4(1H,3H)-pyrimidinedione (L-dT, m.p. 188.5-189 °C) as colorless powder.
NMR (400MHz, $D_2O$, TSP) δ ppm: 1.90 (3H,s,$CH_3$), 6.30 (1H,t,J=7.0Hz,1'-H), 7.66 (1H,s,6-H)

| Anal. (for $C_{10}H_{14}N_2O_5$) | | | |
|---|---|---|---|
| Calcd. | C: 49.58%; | H: 5.83%; | N: 11.57% |
| Found | C: 49.34%; | H: 5.78%; | N: 11.44% |

$[\alpha]_D^{25}$ - 18.9 ° (c=0.996, $H_2O$)

The above-obtained compound, 6.06 g (25 mmol), was dissloved in dry pyridine after being dried twice by azeotropic distillation using dry pyridine, and 8.89 g (26.3 mmmol) of 4,4'-dimethoxytrityl chloride was added under an argon atmosphere. Stirring was continued for 5 hours at room temperature under an argon atmosphere, and then the solvent was removed by distillation after 2.5 ml of methanol was added. The residue was dissolved in 60 ml of chloroform and washed twice with water, dried over anhydrous sodium sulfate and concentrated, and then dried by azeotropic distillation using dry benzene and dry toluene successively. The residue was dissolved in 167 ml of hot benzene and filtered, and the result was left to cool after 67 ml of n-hexane was added. 1-[2-Deoxy-5-(4,4'-dimethoxytrityl)-beta-L-erythro-pentofuranosyl]-5-methyl-2,4(1H,3H)-pyrimidinedione (10.34 g, 75.9%) was obtained as colorless fine crystals (m.p. 127-120 °C).
NMR (400MHz, $CDCl_3$, TMS) δ ppm: 1.47 (3H,s,$CH_3$), 3.79 (6H,s,$OCH_3$ × 2), 6.42 (1H,dd,J=5.8, 7.7Hz,1'-H), 7.58 (1H,s,6-H), 8.70 (1H,s,NH)

The above-obtained DMTr-L-dT 1.09 g (2 mmol) was dried by azeotropic distillation using dry benzene, dry toluene, and dry THF successively. To the solution dissolved in 10 ml of dry THF, 1.4 ml (8 mmol) of diisopropylethylamine and 0.9 ml (4 mmol) of beta-cyanoethylmonochloro-N,N-diisopropylaminophosphoramidite was successively added under an argon atmosphere. After stirring was continued for 35 minutes at room temperature, complete consumption of the starting material was observed. Salts formed were removed by filtration under argon atmosphere, and the residue was dissolved in 100 ml of argon-saturated ethyl acetate after concentration. The solution was washed with ice water and twice with chilled saturated aqueous sodium chloride, and then dried over anhydrous sodium sulfate. After concentration, the residue was subjected to silica gel column chromatography and eluted with ethyl acetate : dichloromethane (1:1), and the fractions at about Rf 0.73 were collected. By removing the solvent by evaporation and drying by azeotropic distillation using dry toluene, 3'-O-cyanoethyl-N,N-diisopropylphosphoramidite of 1-[2-deoxy-5-(4,4'-dimethoxytrityl)-beta-L-erythro-pentofuranosyl]-5-methyl-2,4(1H,3H)-pyrimidinedione 1.14 g (76.5%) was obtained as a colorless amorphous product (a mixture of phosphorous diastereomers).
NMR (400MHz, $CDCl_3$, TMS) δ ppm: 1.04-1.17 (12H,m,isopropyl $CH_3$), 1.43 (3H,m,5-$CH_3$), 6.39 (1H,m,1'-H), 8.34 (1H,bs,NH)

Example 4: 3'-O-cyanoethyl-N,N-diisopropylphosphoramidite of 6-benzoylamino-1-[2-deoxy-5-(4,4'-dimethoxytrityl)-beta-L-erythro-pentofuranosyl]-2(1H)-pyrimidinone

A mixture of 7.85 g (70 mmol) of uracil, 18.3 ml (88 mmol) of hexamethyldisilazane, and 2 ml of trimethylsilyl chloride was refluxed for 2 hours. The mixture was then distilled to afford 14.5 g (80.8%) of colorless oil (65-67 °C /0.65 mmHg). To the mixture of 6.92 g of the above-obtained compound, 1.17 g (8.4 mmol) of para-nitrophenol, and 80 ml of dry dichloromethane, 9.35 g (24 mmol) of 1-chloro-2-deoxy-3,5-bis-O-(4-methoxybenzoyl)-alpha-L-erythro-pentofuranose was added under an argon atmosphere and stirring was continued overnight (20 hours) at room temperature. A small volume of ethanol was added to the reaction mixture to decompose excess TMS-compound, and insoluble precipitate was removed by filtration using celite. Ethanol (50%) was added to the residue obtained by concentrating the filtrate for washing, and crystals were collected by filtration and dried to give 12.7 g of colorless powder. Recrystallization from 45 ml of ethyl acetate gave 1-[3,5-bis-O-(4-methylbenzoyl)-2-deoxy-beta-L-erythro-pentofuranosyl]-2,4(1H,3H)-pyrimidinedione as colorless needles (m.p. 215-216).
NMR (400MHz, $CDCl_3$, TMS) δ ppm: 2.43, 2.44 (3H,s,$CH_3$), 6.41 (1H,dd,J=5.5, 8.4Hz, 1'-H), 8.75 (1H,s,NH)

| Anal. (for $C_{25}H_{24}N_2O_7$) | | | |
|---|---|---|---|
| Calcd. | C: 64.64%; | H: 5.21%; | N: 6.03% |
| Found | C: 64.43%; | H: 5.20%; | N: 5.92% |

$[\alpha]_D^{25}$ + 46.3 ° (c = 0.736, chloroform)

The above-obtained compound, 7.98 g (17.2 mmol), was suspended in 150 ml of dioxane and refluxed for 2 hours after 4.17 g (0.6 equivalent, 10.3 mmol) of Lowesson's Reagent was added. The reaction mixture was cooled and filtered to remove insoluble materials and then the filtrate was concentrated. The residue triturated using 20 ml of ethanol was collected by filtration, washed with a small amount of ethanol, and dried to afford 7.69 g (93.0%) of fine yellow crystals. Recrystallization from ethanol gave 1-[3,5-bis-O-(4-methylbenzoyl)-2-deoxy-beta-L-erythro-pentofuranosyl]-3,4-dihydro-4-thioxo-2(1H)-pyrimidinone having a melting point of 188-189 °C.

NMR (400MHz, $CDCl_3$, TMS) δ ppm: 2.43, 2.44 (3H,s,$CH_3$), 6.34 (1H,dd,J = 3.9, 8.4Hz, 1'-H), 6.26 (1H,d,J = 7.7Hz,6-H), 7.38 (1H,d,J = 7.7Hz,5-H), 9.49 (1H,s,NH)

| Anal. (for $C_{25}H_{24}N_2O_6S$) | | | |
|---|---|---|---|
| Calcd. | C: 62.48%; | H: 5.03%; | N: 5.83% |
| Found | C: 62.34%; | H: 5.00%; | N: 5.71% |

$[\alpha]_D^{25}$ + 74.0 ° (c = 0.572, chloroform)

Dry methanol (200 ml) was saturated with gaseous ammonia under ice cooling, and 7.28 g (15.1 mmol) of the above-obtained compound was added and stirring was continued for 10 hours under heating at 100 °C in a sealed stainless steel cylinder. After cooling, the solvent was removed by evaporation and water was added for drying by azeotropic distillation. The residue was added with 100 ml of water and then successively washed with ethyl acetate and ether. Water was evaporated and ethanol was added to the residue for drying by azeotropic distillation, and then the residue was dissolved in 30 ml of hot methanol and filtered, and the result was left cooling after 100 ml of acetonitrile was added to give 2.35 g (68.5%) of 4-amino-1-(2-deoxy-beta-L-erythro-pentofuranosyl)-2(1H)-pyrimidinone (L-dC, m.p. 196-198).

NMR (400MHz, $D_2O$, TSP) δ ppm: 6.05 (1H,t,J = 7.7Hz, 5-H), 6.27 (1H,t,J = 6.6Hz,1'-H), 7.83 (1H,d,J = 7.4Hz,6-H)

| Anal. (for $C_9H_{13}N_3O_4$) | | | |
|---|---|---|---|
| Calcd.<br>Found | C: 47.57%;<br>C: 47.29%; | H: 5.77%;<br>H: 5.32%; | N: 18.49%<br>N: 18.21% |

$[\alpha]_D^{25}$ − 60.4 ° (c = 0.682, $H_2O$)

The above−obtained compound, 2.27 g (10 mmol), was dried by azeotropic distillation using dry pyridine and dissolved in dry pyridine, to which 6.78 g (20 mmol) of 4,4'−dimethoxytrityl chloride was added under an argon atmosphere. After stirring was continued for 3 hours at room temperature under an argon atmosphere, 6.4 ml (50 mmol) of trimethylsilyl chloride was added using a syringe and stirred for 15 minutes, and then 5.8 ml (50 mmol) of benzoyl chloride was added using a syringe and stirred overnight (20 hours) at room temperature. After ice water (10 ml) was added under ice cooling and stirred for 5 minutes, 20 ml of 29% aqueous ammonia was added and stirring was continued for 30 minutes. The residue obtained by evaporating the solvent was dissolved in 100 ml of chloroform and washed with 5% aqueous sodium bicarbonate solution and twice with water after insoluble materials were removed by filtration. The organic layer was concentrated, subjected to flash column chromatography, and eluted using chloroform and then 2% methanol/chloroform to obtain fractions at Rf = 0.47. A colorless amorphous product obtained by concentrating the fractions was dissolved in 10 ml of dichloromethane and filtered, and then crystallized by adding 150 ml of petroleum ether to give 4.64 g (73.2%) of 4−benzoxyamino−1−[5−O−[bis(4−methoxyphenyl)phenylmethyl]−2−deoxy−beta−L−erythro−pentofuranosyl]−2(1H)−pyrimidinone as white powder (m.p. 118−121 °C).

NMR (400MHz, $CDCl_3$, TMS) δ ppm: 3.80 (6H,s,$OCH_3$ × 2), 6.30 (1H,t,J = 5.5Hz,1'−H), 8.75 (1H,bs,CONH)

The above−obtained DMTr−L−dC 0.634 g (1 mmol) was dried by azeotropic distillation using dry pyridine, dry toluene, and dry THF successively and dissolved in 5 ml of dry THF, and then 0.7 ml (4 mmol) of diisopropylethylamine and 0.45 ml (2 mmol) of beta−cyanoethylmonochloro−N,N−diisopropyoamino−phophoramidite were added successively and stirring was continued for 40 minutes at room temperature. Crystals precipitated were removed by filtration, and the residue was extracted with argon−saturated ethyl acetate and then washed twice with saturated sodium chloride solution. The extract was dried over anhydrous sodium sulfate and concentrated, and then the residue was subjected to silica gel column chromatography and eluted with ethyl acetate : dichloromethane (2:1), and the solvent was removed by evaporation to give 0.41 g (49.2%) of 3'−O−cyanoethyl−N,N−diisopropylphosphoramidite of 6−benzoylamino−1−[2−deoxy−5−(4,4'−dimethoxytrityl)−beta−L−erythropentafuranosyl]−2(1H)−pyrimidinone as a colorless amorphous product after drying by azeotropic distillation using dry toluene (a mixture of phosphorous diastereomers).

NMR (400MHz, $CDCl_3$, TMS) δ ppm: 1.07−1.18 (12H,m,isopropyl $CH_3$), 4.63 (1H,m,3'−H), 6.29 (1H,m,1'−H)

Example 5: 3'−O−cyanoethyl−N,N−diisopropylphosphoramidite of 2−isobutyrilamino−[2−deoxy−5−(4,4'−dimethoxytrityl)−beta−L−erythropentafuranosyl]−1−9,−dihydro−6H−purin−6−one

Sodium hydride, 1.114 g (30 mmol), was washed with dry n−hexane and added to 400 ml of dry acetonitrile. Stirring was continued for 2 hours at 50−60 °C after 3.92 g (23.1 mmol) of 2−amino−6−chloropurine was added. Stirring was then continued overnight at room temperature after 9.41 g (24.2 mmol) of 1−chloro−2−deoxy−3,5−bis−O−(4−methoxybenzoyl)−d−L−erythro−pentofuranose was added. After the reaction mixture was filtrated, a brown viscous crude product was obtained by distillation under a reduced pressure. Purification was carried out by flash chromatography (ethyl acetate : n−hexane = 1:2 to 2:3) to give 5.99 g (ca. 50%) of 2−amino−6−chloro−9−(2−deoxy−3,5−di−O−p−toluoyl−beta−L−erythro−pentofuranosyle)−purine as slightly yellow crystals (m.p. 180−182 °C).

NMR (400MHz, DMSO−$d_6$) δ ppm: 6.40 (1H,t,J = 6.6Hz, 1'−H), 7.01 (2H,s,$NH_2$), 8.34 (1H,s,8−H)

| Anal. (for $C_{26}H_{24}ClN_5O_5$) | | | |
|---|---|---|---|
| Calcd.<br>Found | C: 59.83%;<br>C: 59.85%; | H: 4.63%;<br>H: 4.73%; | N: 13.42%<br>N: 13.20% |

Dry methanol 300 ml was saturated with gaseous ammonia under ice cooling, and stirring was continued overnight at room temperature after 5.99 g of the above-obtained compound was added. Partly crystallized aromatic white or pale yellow oil was obtained by removing the solvent by evaporation. Dichloromethane was added to the oil and the pecipitated crystals were collected by filtration, and were then recrystallized from 100 ml of ethanol to give 1.82 g of 2-amino-6-chloro-9-(2-deoxy-beta-L-erythro-pentofuranosyl)purine (m.p. 156.5-158 °C, docomp.)

NMR (400MHz, DMSO-$d_6$) δ ppm: 6.22 (1H,t,J=7.0Hz, 1'-H), 6.95 (2H,s,$NH_2$), 8.34 (1H,s,8-H)

The above-obtained compound, 3.14 g (11.0 mmol), was added to the mixture of 2N-aqueous potassium hydroxide (190 mmol) and 95 ml of dioxane and refluxed using an oil bath at 110-120 °C. The starting material was dissolved upon heating and the reaction mixture became dark brown. The solvent was evaporated to reduce the mixture to half volume after 13 hours. After 450 ml of purified water was added, the solution was neutralized to pH 6-7 using Dowex-50 ($H^+$). The color of the solution suddenly became light at the neutral point. The resin was removed and the solvent was removed by evaporation to give partly crystallized white or brown viscous oil. The crystals were collected by filtration and washed with ethanol to give 1.39 g (yield 44%) of 2-amino-9-(2'-deoxy-beta-L-erythro-pentofuranosyl)purin-6-one (L-dG) as white-brown crystals (m.p. not less than 300 °C).

NMR (400MHz, DMSO-$d_6$) δ ppm: 6.11 (1H,t,J=6.2, 8.1Hz, 1'-H), 6.46 (2H,s,$NH_2$), 7.91 (1H,s,8-H), 10.56 (1H,s,CONH)

The above-obtained compound, 3.064 g (10.7 mmol), was dried by azeotropic distillation using dry dimethylformamide. Stirring was continued under an argon atmosphere for 5 hours at room temperature after 30.1 ml of dry dimethylformamide and 15 ml (7.3 mmol) of 1,1,1,3,3,3-hexamethyldisilazane were added. Dry pyridine 30.1 ml and isobutyric anhydride 30.1 ml were added and stirring was continued overnight at room temperature. After addition of 64 ml of dry methanol, the reaction mixture was left at room temperature for 3 hours, removed of the solvent by distillation until the volume became 20-30 ml, added with 170 ml of a mixture of ether/n-hexane (1:1) and thoroughly ice-chilled. The precipitated crystals were collected by filtration to obtain 5.40 g of crude brown crystals, which were recrystallized from the mixture of water (25 ml) and methanol (12 ml) to afford 2.34 g (yield 65%) of 2-isobutyrilamino-9-(2-deoxy-beta-L-erythro-pentofuranosyl'purin-6-one as brownish white crystals (m.p. not less than 300 °C).

NMR (400MHz, DMSO-$d_6$) δ ppm: 1.11, 1.13 (3H,s,$CH_3$ × 2), 6.21 (1H,t,J=7.7Hz,1'-H), 8.24 (1H,s,8-H)

| Anal. (for $C_{14}H_{19}N_5O_5$) | | | |
|---|---|---|---|
| Calcd. | C: 49.85%; | H: 5.68%; | N: 20.76% |
| Found | C: 49.50%; | H: 5.61%; | N: 20.70% |

$[\alpha]_D^{27}$ + 6.93 ° (c=1.01, DMF)

Dry pyridine was added to 2.04 g of the above-obtained compound and dried twice by azeotropic distillation using dry pyridine, and vigorous stirring was continued for 6 hours at room temperature after addition of 93.1 mg (0.784 mmol) of 4-dimethylaminopyridine (10 ml solution in pyridine), 50 ml of dioxane, and 3.845 g (10.7 mmol) of dimethoxytrityl chloride. The reaction mixture was added with 50 ml of 5% aqueous sodium bicarbonate solution and extracted twice with 75 ml of dichloromethane and once with 20 ml of dichloromethane, and then the organic layer was dried over anhydrous sodium sulfate after being washed with saturated sodium chloride solution. By evaporating the solvent, 14.9 g of red-brown crude product was obtained, which was then purified by silica gel column chromatography (ethyl acetate - ethyl acetate : methanol = 9:1) to afford 2.24 g (yield 59%) of 9-[5-O-(4,4'-dimethoxytrityl)-2-deoxy-beta-L-erythro-pentofuranosyl]-2-isobutylamino-purin-6-one as orangish white foamy product.

NMR (400MHz, DMSO-$d_6$) δ ppm: 1.11, 1.13 (3H,s,$CH_3$ × 2), 6.25 (1H,t,J=6.6Hz,1'-H), 6.23-7.32 (m,Ar-H), 8.10 (1H,s,8-H), 11.7, 12.1 (1H,s,CONH× 2)

The above-obtained compound 0.640 g (1.0 mmol) was dried by azeotropic distillation using dry pyridine (twice), dry toluene, and dry THF. After the atmosphere was replaced with argon, 5 ml of dry THF, 0.7 ml (4 mmol) of N,N,N-diisopropylethylamine, and 0.45 ml (20 mmol) of beta-cyanoethylmonochloro-N,N-diisopropylamino phosphoramidite were successively added and stirring was continued for 35 minutes at room temperature. The precipitated crystals were removed by filtration and the residue was dissolved in 50 ml of argon-saturated ethyl acetate and washed twice with ice-chilled saturated aqueous sodium chloride solution, which was then dried over anhydrous sodium sulfate and concentrated. The residue was subjected to silica gel column chromatography and eluted with ethyl acetate : dichloromethane (2:1), and

then the solvent was removed by evaporation to afford 0.722 g of 3'-O-cyanoethyl-N,N-diisopropyl-phosphoramidite of 2-isobutyrilamino-9-[2-deoxy-5-(4,4'-dimethoxytrityl)-beta-erythro-pentofuranosyl]-1,9-dihydro-6H-purin-6-one as a colorless amorphous product (a mixture of phosphorous diastereomers) after drying by azeotropic distillation using dry toluene.
NMR (400MHz, DMSO-$d_6$) $\delta$ ppm: 6.25 (1H,t,J=7.2Hz,1'-H), 8.13 (1H,s,8-H), 11.6 (1H,bs,CONH), 12.1 (1H,bs,CONH)

Example 6: Preparation of $(L-dA)_6$ by solid phase synthesis

A DNA synthesizer equipped with a DPG column bound to 1 µmol of deoxyadenosine was connected with a bottle containing phosphoramidite (250 mg) obtained by Example 2, and six cycles of nucleoside extension reactions were carried out. The product was recovered from the CPG column and des-benzoylated using concentrated aqueous ammonia, which was then purified by HPLC and further des-tritylated using 80% acetic acid. The resulting product was fractionated using HPLC and lyophilized after desalting to obtain 7-mer having D-deoxyadenosine (natural type) at the 5'-end. This product was treated with snake venom phosphodiesterase, fractionated by HPSC and desalted, and then lyophilized to give compound $(L-dA)_6$ (150 µg, 8 OD units). This compound was spectroscopically (NMR and CD spectrum) identical with the compound of Example 1.

In the same manner as describe avove, $(L-dA)_6$, $(L-dT)_{12}$, $(L-dT)_{100}$, $(L-dG)_{12}$, and $(L-dC)_{12}$ were prepared. Furthermore, L-AATACTCATACTCTTC, L-CTTCTCATACTCATAA, and L-TGGCCAAGCT were synthesized in the same manner.

Table 1

| CD Spectrum Data | |
|---|---|
| COMPOUND | nm ( $[\theta]^{20}$ °C ) |
| $(L-dA)_6$ * | 304(0), 270(-6200), 260(0), 250(+17800), 239(0) |
| $(L-dA)_{12}$ * | 293(0), 277(-3560), 261(0), 250(+13300), 240(0) |
| $(L-dT)_{12}$ * | 303(0), 278(-10000), 262(0), 250(+6200), 231(0) |
| $(L-dC)_{12}$ * | 302(0), 275(-20000), 246(0), 230(+9840) |
| $(L-dG)_{12}$ * | 320(0), 265(-32000), 250(0), 241(+14000), 223(0) |
| L-AATACTCATACTCTTC ** | 298(0), 274(-160000), 260(0), 247(+140000), 226(0) |

* Solvent: 10 mM Tris (pH 7.5) - 10 mM $MgCl_2$
** 0.01 M Phosphate buffer (pH 7.0)

Example 7

The interaction of $(L-dG)_{12}$ obtained by Example 6 with poly C was studied by measuring the CD spectrum. The interaction between the two compounds can be demonstrated by the presence of the difference between the measured CD value (m ° ) of the mixture and the calculated CD value (° ) as they do not interact each other and the difference disappears at an elevated temperature. It is apparent from the CD spectra data (measured at 0.09 mM in 10 mM Tris-HCl/10 mM $MgCl_2$ buffer; cell length: 1 mm; mdeg/284 nm) that they strongly interact with each other.

Table 2

| CD Spectrum of $(L-dG)_{12}$ | | |
|---|---|---|
| Oligomer | 0 °C | 80 °C |
| $(L-dG)_{12}$ | -0.56 | -1.09 |
| Poly C | 4.46 | 2.11 |
| 1:1 mixture (calc.) | 1.95 | 0.51 |
| 1:1 mixture (found) | 1.31 | 0.47 |
| difference | -0.64 | -0.04 |

Example 8: Preparation of $(LD-dA)_{12}$ by solid phase synthesis

Natural $(D-dA)_{12}$, $(L-dA)_{12}$ which is the enantiomeric DNA of the present invention, $[(L-dAp)-dA]_6$ which is the meso-type DNA of the present invention containing D-dA and L-dA alternately (hexamer of L-deoxyadenyl-D-deoxyadenylic acid: hereinafter referred to as $(LD-dA)_{12}$) were prepared with an automatic DNA synthesizer (Applied Biosystems) using the above-obtained N-benzoyl-L-deoxyadenosine-cyanoethylphophoramidite and commercially available N-benzoyl-D-deoxyadenosine-cyanoethylphosphoramidite as reagents, and purified by high perfomance liquid chromatography. $(D-dA)_{12}$ exhibited a CD-spectrum having the same shape but the opposite sign to that of $(L-dA)_{12}$. However, $(LD-dA)_{12}$ exhibited a flat spectrum. The molar extinction coefficient of the dodecamer was estimated as $\epsilon_{260}$ = 9,700 using the value obtained with the corresponding monomers after enzymatic hydrolysis. In addition, $(LD-dT)_{12}$ was prepared in the same manner as described above.

Example 9: Interaction between homopolynucleotide and L-oligodeoxyribonucleotide of the present invention (mixing curve)

Solutions (0.1 mM) of $(L-dA)_6$, poly U, and poly dT were prepared (10 mM Tris, pH 7.4-10 mM $MgCl_2$), and mixed solutions were prepared in various volume ratios. The solutions were heated for 10 minutes at 80°C and successively cooled at 0 °C for 2 hours. Absorbance [A] in the ultraviolet region was measured at 0 °C. As a reference, natural deoxyadenosine $(D-dA)_6$ was treated in the same manner. Fig. 1 shows the mixing curve of $(L-dA)_6$ with poly U and poly L-A with poly dT, and Fig. 2 shows the mixing curve of $(D-dA)_6$ with poly U and poly (A) with poly dT.

The results shown in Figs.1 and 2 demonstrate that $(L-dA)_6$ that is the L-oligodeoxyribonucleotide of the present invention does not interact with poly dT, while it has the same degree of interaction with poly U as $(D-dA)_6$. The hypochromicity at the turning point was about 20%. Accordingly, it is apparent that $(L-dA)_6$ binds only to RNA. The mixing curve of $(L-dA)_{12}$ was obtained in a similar manner (0 °C). From the results shown in Fig. 3, it is apparent that $(L-dA)_{12}$ interacts with both with DNA and RNA at a low temperature.

Example 10: Interaction of a homopolynucleotide with LD-oligodeoxyribonucleotide of the present invention (mixing curve)

The interaction of $(LD-dA)_{12}$ with poly U or poly dT was studied in the same manner as in Example 9. From the results shown in Fig. 4, it is apparent that $(LD-dA)_{12}$ of the present invention interacts with both poly U and ply dT in the same manner as natural $(D-dA)_{12}$. Maximum hypochromicity was about 30%. As $(LD-dA)_{12}$ did not show hypochromicity with other homopolynucleotides, the interaction of $(LD-dA)_{12}$ with homopolynucleotides are clearly complementary sequence-specific.

Example 11: Interaction of a homopolynucleotide with L-oligodeoxyribonucleotide of the present invention (melting curve)

A solution of $(L-dA)_6$ and poly U (1:2) (0.1 mM in 10 mM Tris, pH 7.4-10 mM $MgCl_2$), and a solution of $(L-dA)_6$ and poly dT (1:1) (0.1 mM in 10 mM Tris, pH 7.4-10 mM $MgCl_2$) were heated at 80 °C for 10 minutes, and then left at 4 °C overnight and 0°C for 2 hours, whereafter changes in absorbance [A] at 260 nm were measured while gradually heating an ultraviolet spectroscopic cell with an incubator. As a

reference, natural $(D-dA)_6$ was treated in the same manner.

Fig. 5 and Fig. 6 show the changes in absorbance-temperature of each complex. From the results shown in Fig. 5, it can be understood that the complex of $(L-dA)_6$ that is the L-oligodeoxyribonucleotide of the present invention and poly U exhibits sharp increase of absorbance from 30 °C. Tm value was 32.5°C. On the other hand, the mixture of $(L-dA)_6$ and poly dT does not exhibit such phenomonon. The results with reference to the mixture of $(D-dA)_6$ andpoly U or poly dT are shown in Fig. 6.

Example 12: Interaction of a homopolynucleotide with LD-oligodeoxyribonucleotide of the present invention (melting curve)

Melting curves were obtained using $(LD-dA)_{12}$ and poly U or poly dT in the same manner as Example 11. The complexes of $(LD-dA)_{12}$ with poly U and $(LD-dA)_{12}$ with poly dT exhibit single melting points at 61.5 °C and 50.5 °C, respectively (see, Figs. 7 and 8). It is apparent from these results that $(LD-dA)_{12}$ of the present invention interacts with both poly U and poly dT. The melting points of the complexes are summarized in Table 3 below which were obtained under the same conditions.

Table 3

| Melting points of the complex formed by dodecamer and homopolynucleotide | | |
|---|---|---|
| Oligomer | Poly U | Poly dT |
| $(D-dA)_{12}$ | 72.5 | 48.0 and 74.0 |
| $(LD-dA)_{12}$ | 61.5 | 50.5 |
| $(L-dA)_{12}$ | 53.5 and 68.5 | 15.5 and 67.5 |

Example 13: Hydrolysis by phosphodiesterase

Bovine spleen phosphodiesterase (0.37 units) was added to $(L-dA)_6$ (0.334 µmol in ammonium acetate buffer, pH 6.5) and changes in absorbance were measured at 260 nm at 37 °C. From the results summarized in Fig. 9, it is apparent that the L-oligodeoxyribonucleotide of the present invention is hardly hydrolyzed enzymatically.

Example 14: Hydrolysis by phosphodiesterase

$(D-dA)_{12}$ prepared in Example 7, $(L-dA)_{12}$. and $(LD-dA)_{12}$ were treated by bovine spleen phosphodiesterase (BSP, 25 U/ml) in ammonium acetate buffer (pH 6.5) or snake venom phosphodiesterase (SVP, 2.5 U/ml) in ammonium bicarbonate buffer (pH 9) and the degree of hydrolysis was determined by measuring absorbance at 260 nm at 37 °C. The results obtained are shown in Fig. 10. $(D-dA)_{12}$ was completely hydrolyzed by bovine spleen phosphodiesterase (BSP) after 40 minutes, while $(LD-dA)_{12}$ was not hydrolyzed by bovine spleen phosphodiesterase (BSP) and hardly hydrolyzed by snake venom phosphodiesterase (SVP).

In addition, after $(LD-dA)_{12}$ was treated with snake venom phosphodiesterase for 24 hours, analysis by high performance liquid chromatography was carried out to reveal that $(LD-dA)_{12}$ was partially hydrolyzed to give 4 to 10-mer represented by $(LD-dA)_n$ wherein n is an integer from 4 to 10.

Industrial Applicability

The oligodeoxyribonucleotides of the present invention are stable to phosphodiesterase in a living body, and they are useful as antisense DNAs since they specifically bind to a complementary nucleic acid. In particular, they are useful for medication or biological research, and are expected to be useful as, for example, anti-viral agent.

## Claims

**1.** An oligodeoxynucleotide comprising an oligonucleotide formed by linking the 5'-phosphoric acid ester of a nucleoside that comprises 2-deoxy-L-erythro-pentose and a nucleic acid base linked together

in beta-configuration by the 3'→5' phosphodiester linkage.

2. An oligodeoxynucleotide comprising an oligonucleotide formed by linking the 5'-phosphoric acid ester of a nucleoside that comprises 2-deoxy-L-erythro-pentose and a nucleic acid base linked together in beta-configuration by the 3'→5' phosphodiester linkage which specifically binds to a natural oligonucleotide comprising a complementary sequence to that of the oligonucleotide.

3. The oligodeoxyribonucleotide according to claim 2 wherein the natural oligonucleotide comprising a complementary sequence is RNA.

4. The oligodeoxyribonucleotide according to claim 3 which does not bind substantially to a natural DNA comprising a complementary sequence.

5. An oligodeoxynucleotide comprising an oligonucleotide formed by alternately linking the 5'-phosphoric acid ester of a nucleoside that comprises 2-deoxy-D-ribose and a nucleic acid base linked together in beta-configuration with the 5'-phosphoric acid ester of a nucleoside that comprises 2-deoxy-L-erythro-pentose and a nucleic acid base linked together in beta-configuration by the 3'→5'phosphodiester linkage.

6. An oligodeoxynucleotide comprising an oligonucleotide formed by alternately linking the 5'-phosphoric acid ester of a nucleoside that comprises 2-deoxy-D-ribose and a nucleic acid base linked together in beta-configuration with the 5'-phosphoric acid ester of a nucleoside that comprises 2-deoxy-L-erythro-pentose and a nucleic acid base linked together in beta-configuration by the 3'→5'phosphodiester linkage which specifically binds to a natural oligonucleotide comprising a complementary sequence to that of the oligonucleotide.

7. The oligodeoxynucleotide according to claim 6 which binds to a natural RNA comprising a complementary sequence and to a natural DNA comprising a complementary sequence.

8. A phosphoramidite derivative represented by the following formula:

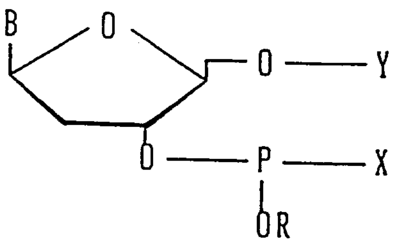

wherein, B represents a nucleic acid base which may optionally be protected, R represents cyanoethyl group or a lower alkyl group, X represents an amino group which may optionally be protected, and Y represents a protective group for hydroxyl group.

9. 3'-O-Cyanoethyl-N,N-diisopropylphosphoramidite derivative of 6-benzoylamino-9-[2-deoxy-5-(4,4'-dimethoxytrityl)-beta-L-erythro-pentofuranosyl]-9H-purine.

10. 3'-O-Cyanoethyl-N,N-diisopropylphosphoramidite derivative of 1-[2-deoxy-5-(4,4'-dimethoxytrityl)-beta-L-erythro-pentofuranosyl]-5-methyl-2,4(1H,3H)-pyrimidinedione.

11. 3'-O-cyanoethyl-N,N-diisopropylphosphoramidite derivative of 6-benzoylamino-1-[2-deoxy-5-(4,4'-dimethoxytrityl)-beta-L-erythro-pentofuranosyl]-2(1H)-pyrimidinone.

12. 3'-O-cyanoethyl-N,N-diisopropylphosphoramidite derivative of 2-isobutyrylamino-9-[2-deoxy-5-(4,4'-dimethoxytrityl)-beta-L-erythro-pentofuranosyl]-1,9-dihydro-6H-purine-6-one.

**13.** 3'-O-cyanoethyl-N,N-diisopropylphosphoramidite derivative of 6-isobutyrylamino-1-[2-deoxy-5-(4,4'-dimethoxytrityl)-beta-L-erythro-pentofuranosyl]-2(1H)-pyrimidinone.

**14.** 3'-O-cyanoethyl-N,N-diisopropylphosphoramidite derivative of 6-(N,N-dimethylamidino)-9-[2-deoxy-5-(4,4'-dimethoxytrityl)-beta-L-erythro-pentofuranosyl]-9H-purine.

**15.** 3'-O-cyanoethyl-N,N-diisopropylphosphoramidite derivative of 2-(N,N-dimethylamidino)-9-[2-deoxy-5-(4,4'-dimethoxytrityl)-beta-L-erythro-pentofuranosyl]-1,9-dihydro-6H-purine-6-one.

**16.** A process for preparing an oligodeoxynucleotide comprising an oligonucleotide formed by linking the 5'-phosphoric acid ester of a nucleoside that comprises 2-deoxy-L-erythro-pentose and a nucleic acid base linked together in beta-configuration by the 3'→5' phosphodiester linkage, which comprises the steps of:

(a) nucleotide bond forming step comprising a first step of forming nucleotide bond by reacting the L-phosphoramidite derivative according to claim 8 with the terminal nucleoside of a deoxyribonucleoside immmobilized to a solid phase or an oligodeoxyribonucleotide immobilized to a solid phase; a second step of acylating 5'-hydroxyl group, and a third step of oxidizing the phosphorus;
(b) nucleotide extension step comprising a step of conducting from the first step to the third step of the above-described step (a) using the L-phosphoramidite derivative according to claim 8 after the 5'-protective group of immobilized nucleotide obtained in the above step (a) is removed;
(c) step of repeating the above step (b), if desired; and
(d) step of removing protective groups comprising the steps of removing the protective groups for the phosphoramidite moiety of the immobilized protected oligodeoxyribonucleotide obtained above and carrying out the cleavage from the solid phase; removing the protective groups for the base moiety of the oligodeoxyribonucleotide; and removing the 5'-protective group.

**17.** The process according to claim 16 wherein the deoxyribonucleoside immmobilized to a solid phase is a natural nucleoside.

**18.** The process according to claim 16 wherein the oligodeoxyribonucleotide immmobilized to a solid phase is a natural nucleoside.

**19.** The process according to any one of claims 17 and 18, wherein the process further comprises a step of removing a natural oligodeoxynucleotide or a natural nucleoside by treating the oligodeoxyribonucleotide obtained with a phophodiesterase.

**20.** A process for preparing an oligodeoxynucleotide comprising an oligonucleotide formed by linking alternately linking the 5'-phosphoric acid ester of a nucleoside that comprises 2-deoxy-D-ribose and a nucleic acid base linked together in beta-configuration with the 5'-phosphoric acid ester of a nucleoside that comprises 2-deoxy-L-erythro-pentose and a nucleic acid base linked together in beta-configuration by the 3'→5'phosphodiester linkage, which comprises the steps of:

(a) nucleotide bond forming step comprising a first step of forming nucleotide bond by reacting the L-phosphoramidite derivative according to claim 8 with the terminal nucleoside of a deoxyribonucleoside immmobilized to a solid phase or an oligodeoxyribonucleotide immobilized to a solid phase; a second step of acylating 5'-hydroxyl group, and a third step of oxidizing the phosphorus;
(b) nucleotide extension process comprising a step of conducting from the first step to the third step of the above-described step (a) using a natural type D-phosphoramidite derivative after the 5'-protective group of immobilized nucleotide obtained in the above process (a) is removed, followed by conducting from the first step to the third step of the above-described step (a) using the L-phosphoramidite according to claim 8;
(c) step of repeating the above step (b), if desired; and
(d) step of removing protective groups comprising the steps of removing the protective groups for the phosphoramidite moiety of the immobilized protected oligodeoxyribonucleotide obtained above and carrying out the cleavage from the solid phase; removing the protective groups for the base moiety of the oligodeoxyribonucleotide; and removing the 5'-protective group.

**21.** The process according to claim 20 wherein the deoxyribonucleoside immmobilized to a solid phase is a natural nucleoside.

**22.** The process according to claim 20 wherein the oligodeoxyribonucleotide immmobilized to a solid phase is a natural nucleoside.

**23.** The process according to any one of claims 21 and 22, wherein the process further comprises the step of removing a natural oligodeoxynucleotide or a natural nucleoside by treating the oligodeoxyribonucleotide obtained with a phophodiesterase.

**24.** A gene expression regulating agent which comprises an oligodeoxynucleotide comprising an oligonucleotide formed by linking the 5'-phosphoric acid ester of a nucleoside that comprises 2-deoxy-L-erythro-pentose and a nucleic acid base linked together in beta-configuration by the 3' →5' phosphodiester linkage.

**25.** A gene expression regulating agent which comprises an oligodeoxynucleotide comprising an oligonucleotide formed by linking alternately linking the 5'-phosphoric acid ester of a nucleoside that comprises 2-deoxy-D-ribose and a nucleic acid base linked together in beta-configuration with the 5'-phosphoric acid ester of a nucleoside that comprises 2-deoxy-L-erythro-pentose and a nucleic acid base linked together in beta-configuration by the 3' →5'phosphodiester linkage.

**26.** An antisense oligodeoxynucleotide comprising an oligonucleotide formed by linking the 5'-phosphoric acid ester of a nucleoside that comprises 2-deoxy-L-erythro-pentose and a nucleic acid base linked together in beta-configuration by the 3'→5' phosphodiester linkage.

**27.** An antisense oligodeoxynucleotide comprising an oligonucleotide formed by linking alternately linking the 5'-phosphoric acid ester of a nucleoside that comprises 2-deoxy-D-ribose and a nucleic acid base linked together in beta-configuration with the 5'-phosphoric acid ester of a nucleoside that comprises 2-deoxy-L-erythro-pentose and a nucleic acid base linked together in beta-configuration by the 3'→5'phosphodiester linkage.

FIG. 1

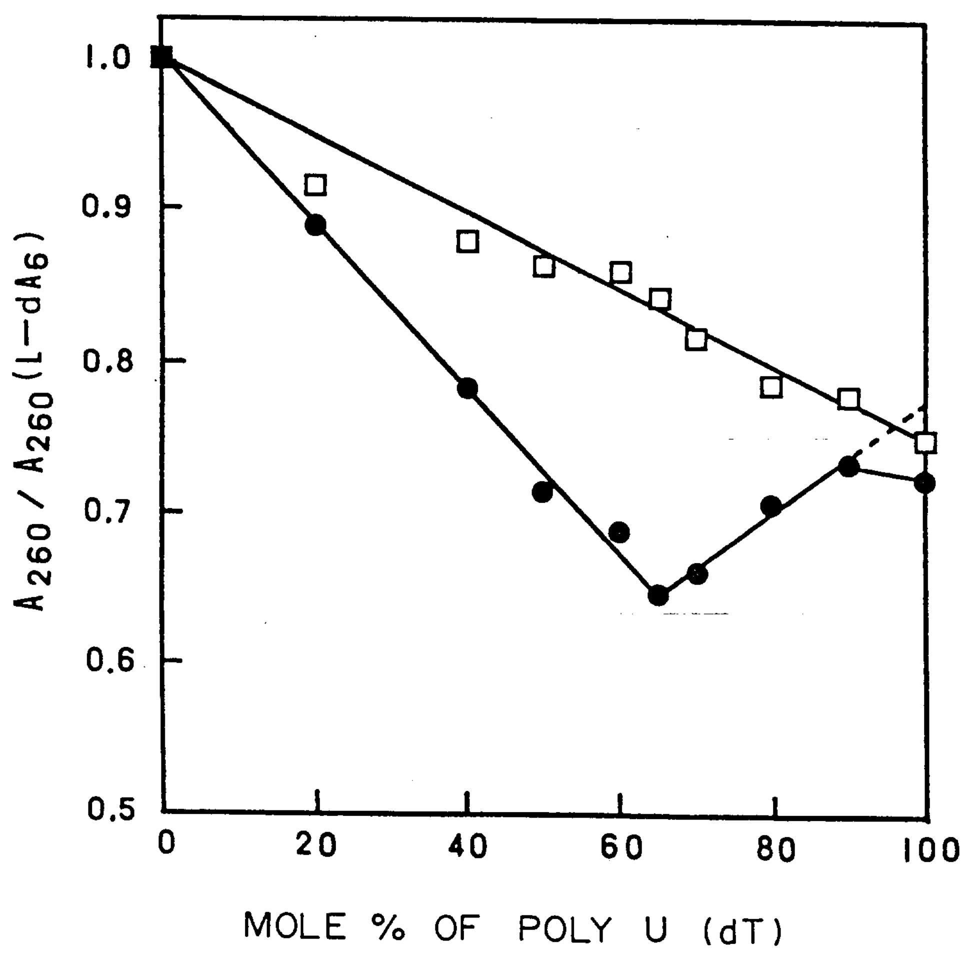

FIG. 2

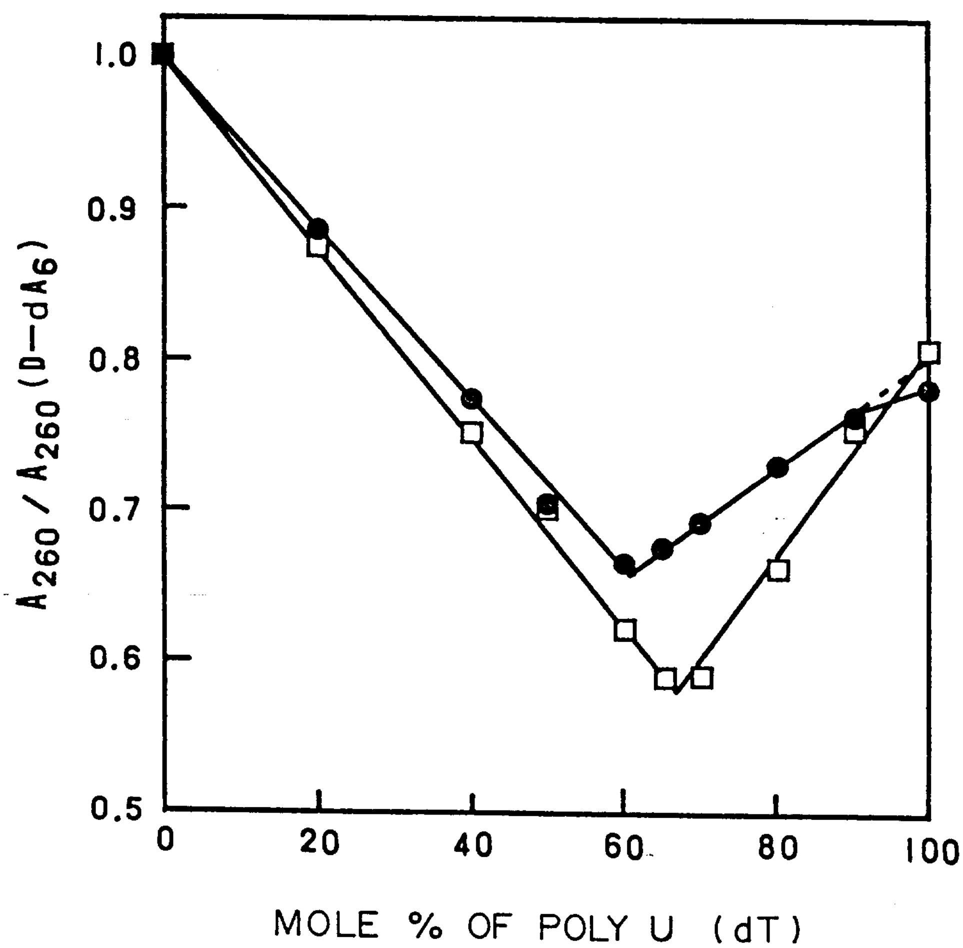

FIG. 3

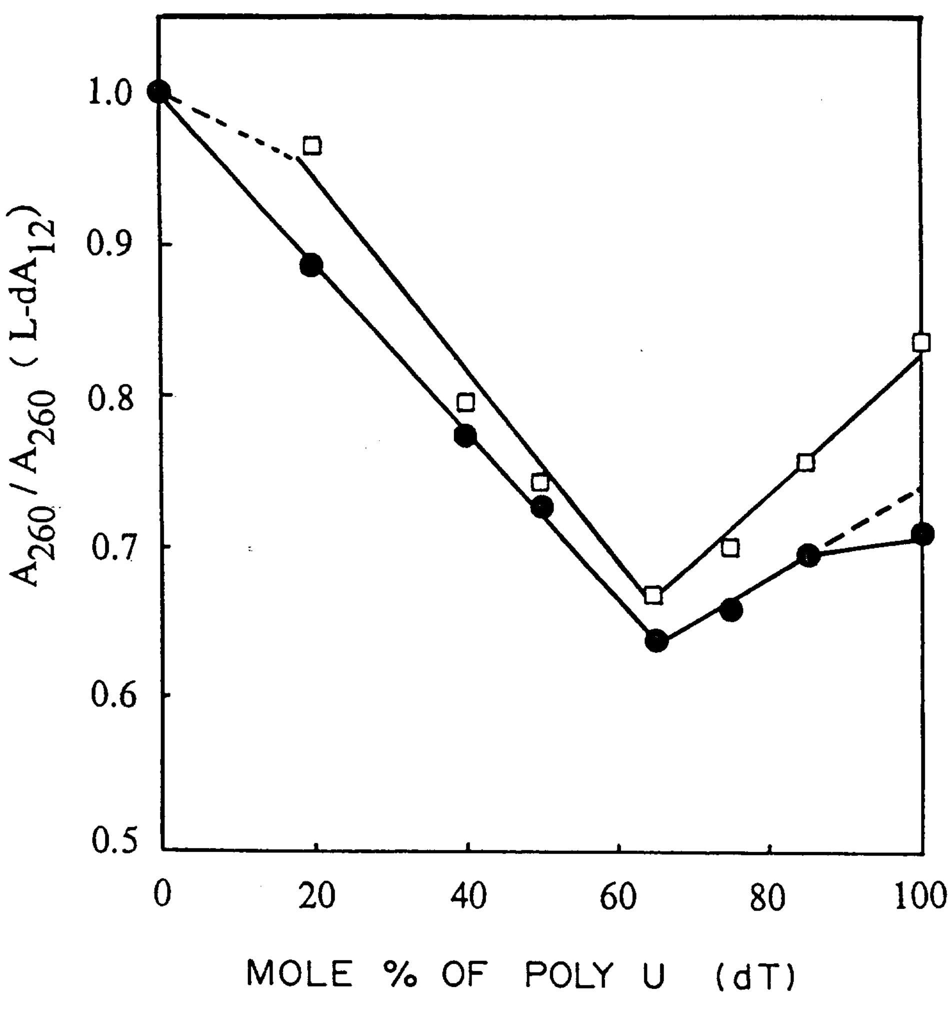
1.0
0.9
0.8
0.7
0.6
0.5
A260 / A260 ( L-dA12 )
0
20
40
60
80
100
MOLE % OF POLY U (dT)

FIG. 4

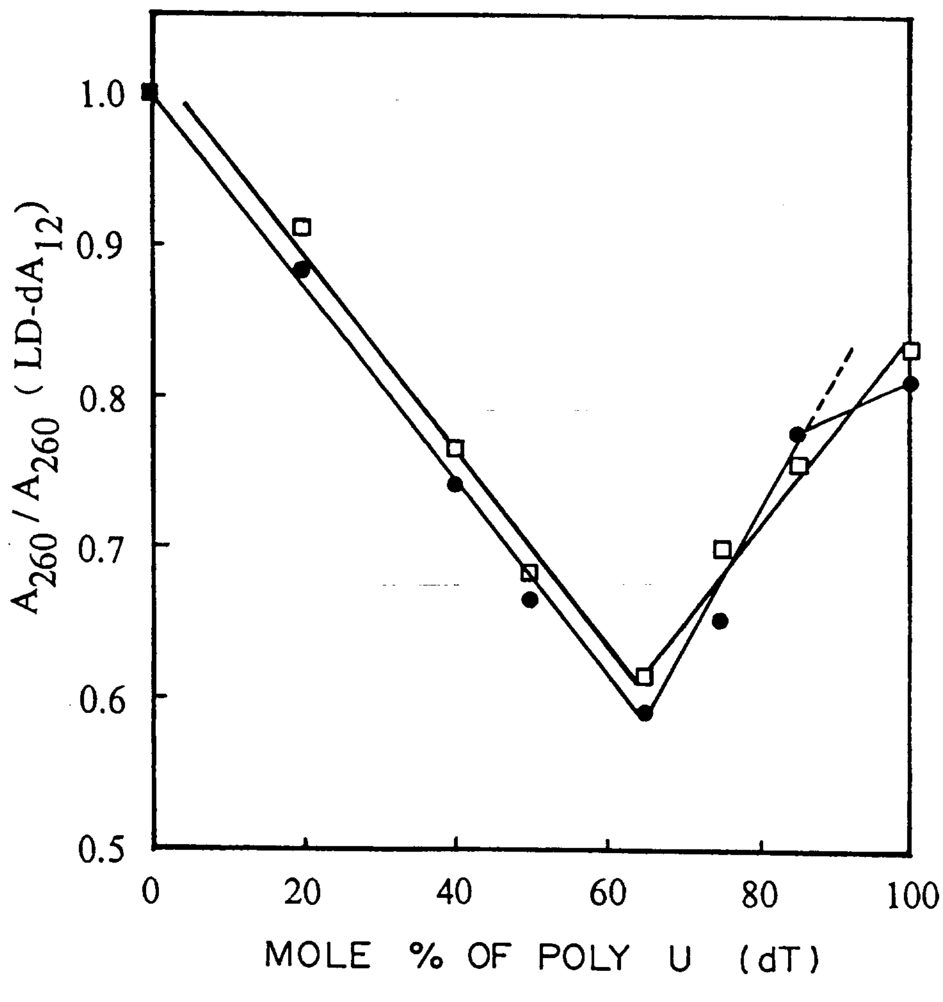
1.0
0.9
0.8
0.7
0.6
0.5
$A_{260} / A_{260}$ ( LD-d$A_{12}$)
0
20
40
60
80
100
MOLE % OF POLY U (dT)

FIG. 5

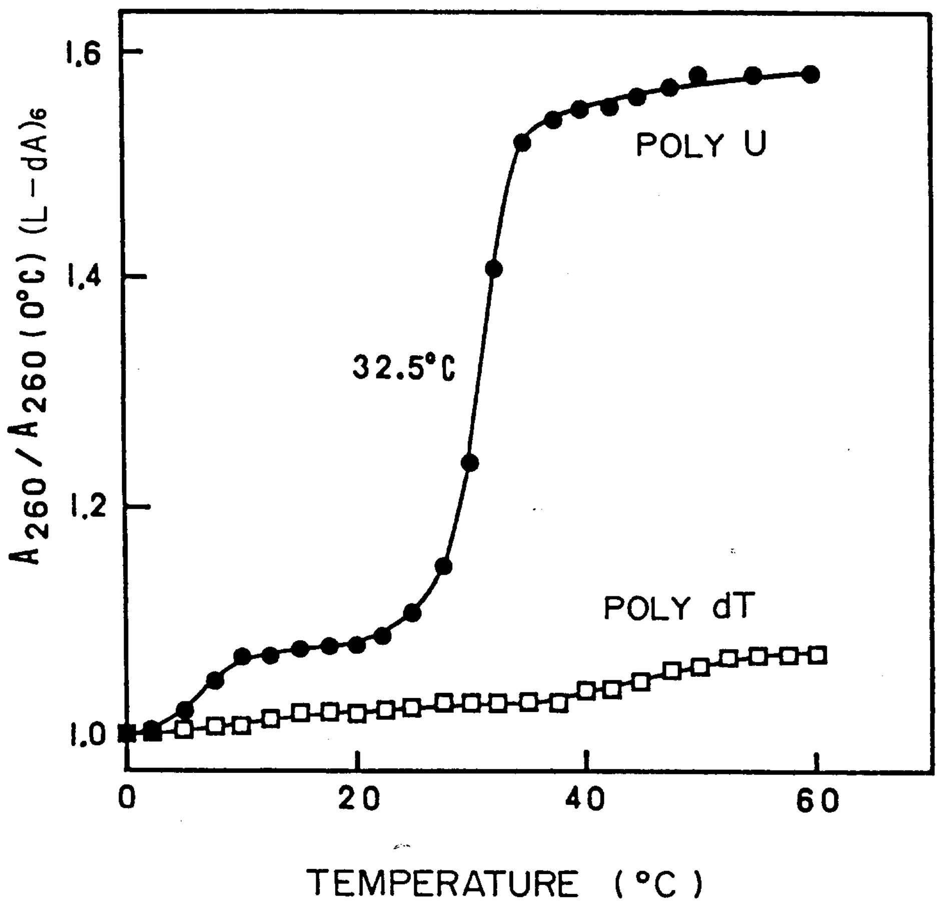
1.6
1.4
1.2
1.0
$A_{260}/A_{260}(0°C)$ (L−dA)6
POLY U
32.5°C
POLY dT
0
20
40
60
TEMPERATURE (°C)

FIG. 6

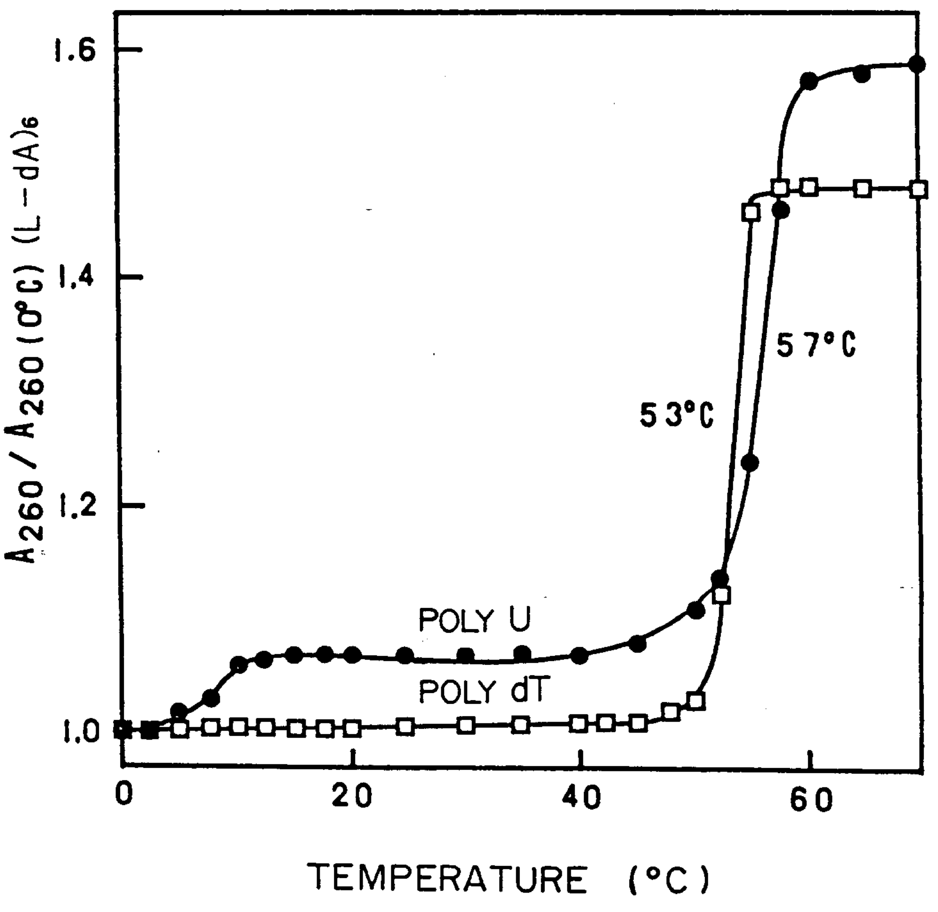
1.6
1.4
1.2
1.0
$A_{260} / A_{260}$ (0°C) (L-dA)6
0
20
40
60
TEMPERATURE (°C)
POLY U
POLY dT
53°C
57°C

FIG. 7

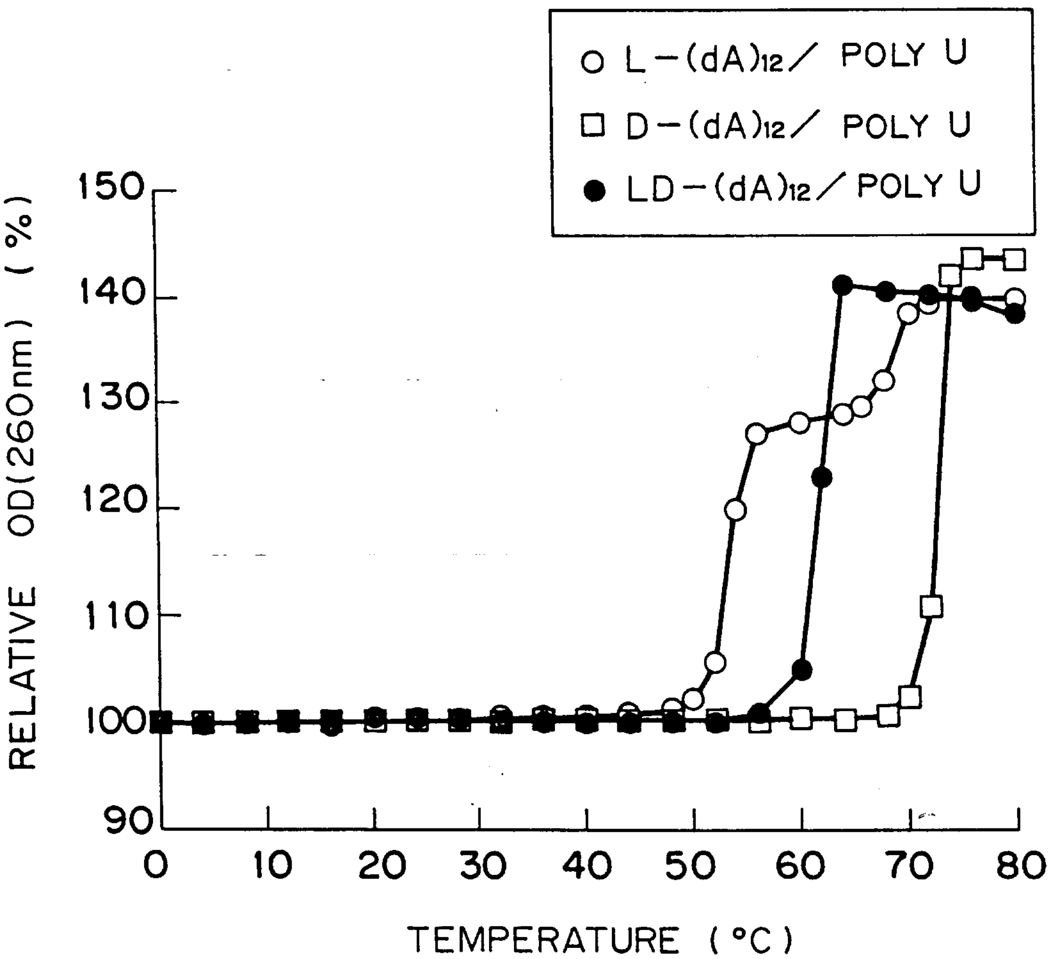
○ L−(dA)12/ POLY U
□ D−(dA)12/ POLY U
● LD−(dA)12/ POLY U
RELATIVE OD(260nm) (%)
150
140
130
120
110
100
90
0
10
20
30
40
50
60
70
80
TEMPERATURE (°C)

FIG. 8

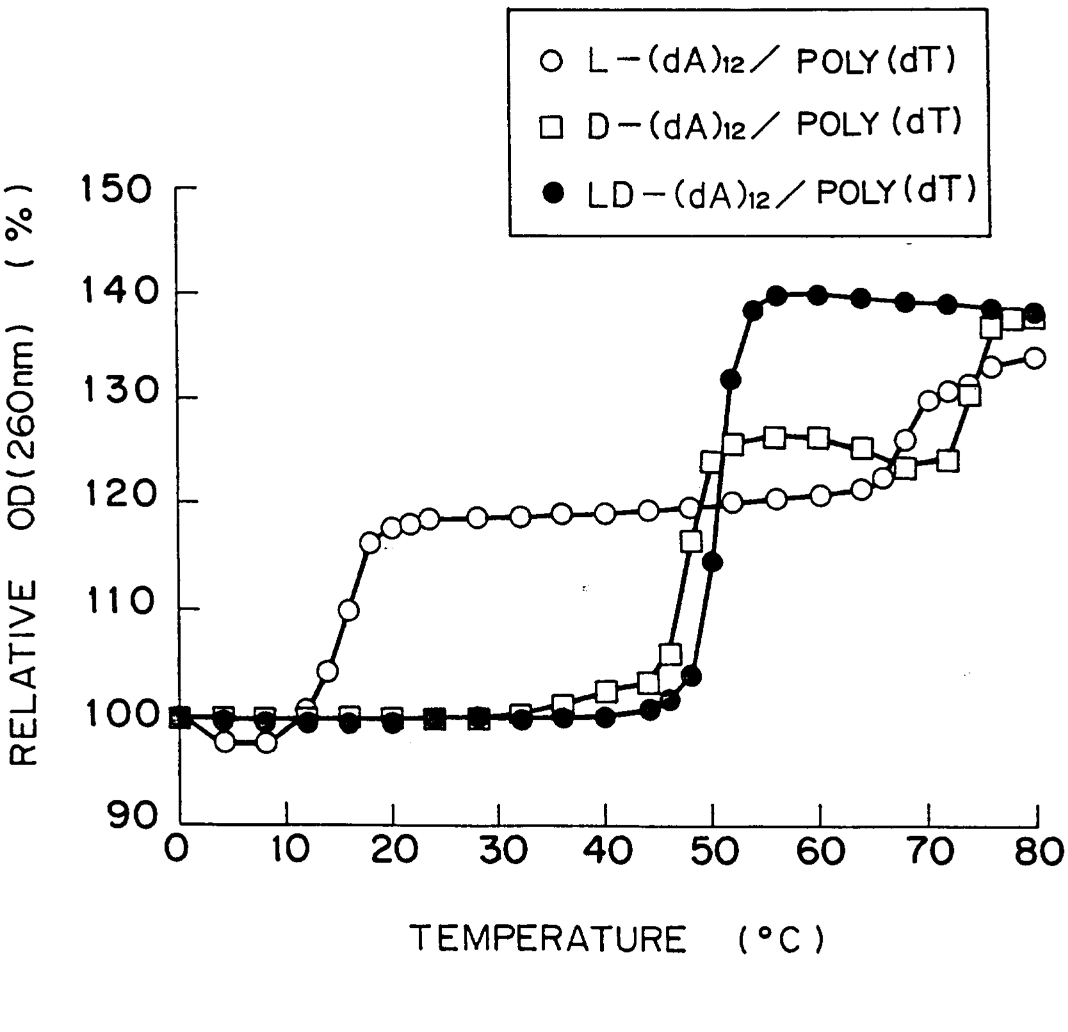
○ L-(dA)12/ POLY(dT)
□ D-(dA)12/ POLY(dT)
● LD-(dA)12/ POLY(dT)
RELATIVE OD(260nm) (%)
150
140
130
120
110
100
90
0
10
20
30
40
50
60
70
80
TEMPERATURE (°C)

FIG. 9

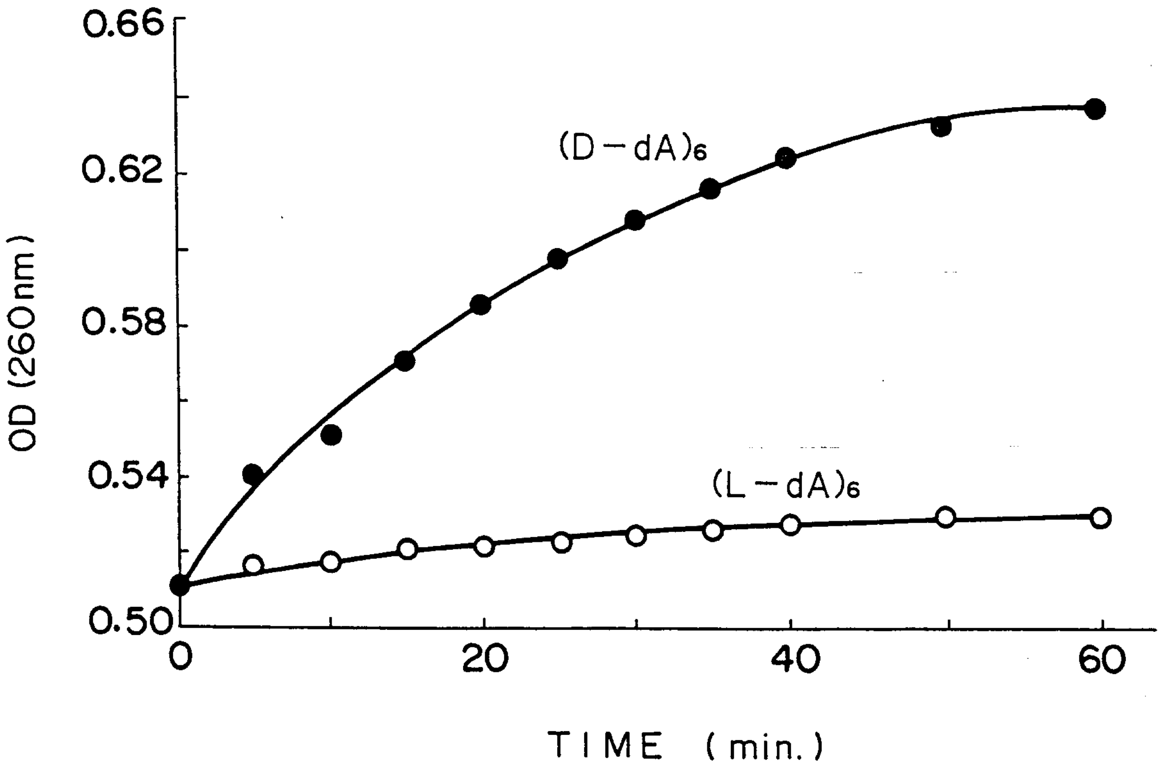

FIG. 10

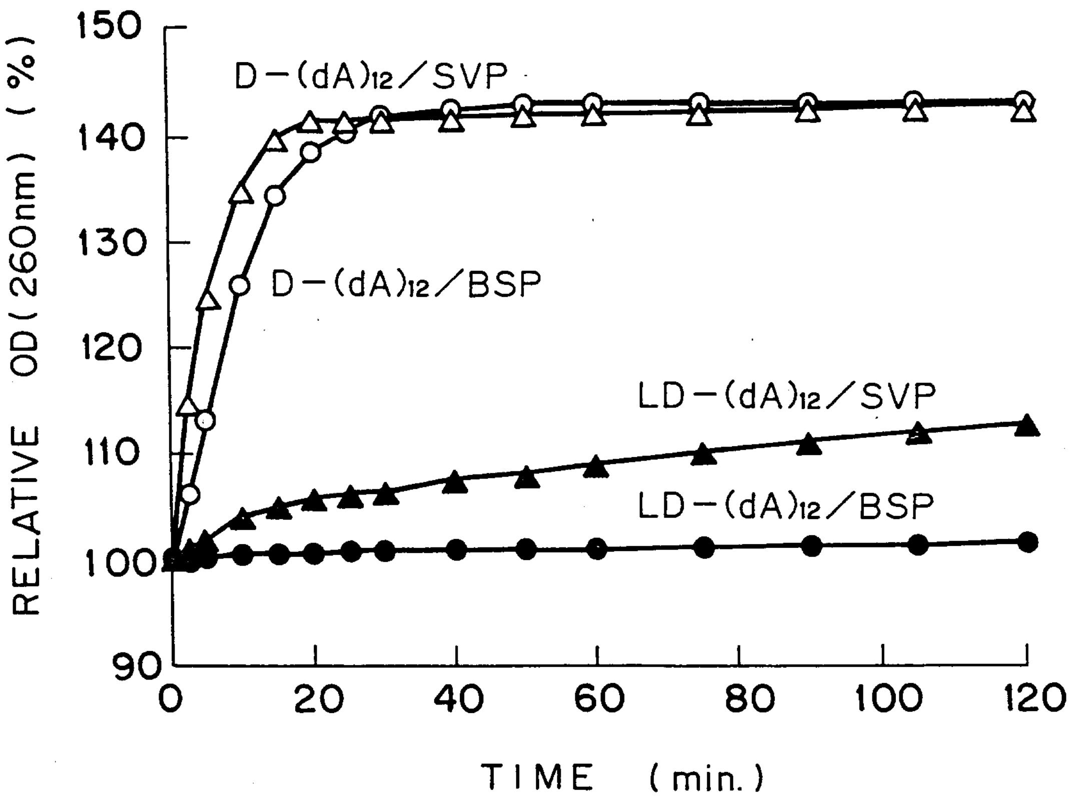
150
140
130
120
110
100
90
RELATIVE OD(260nm) (%)
D−(dA)12/SVP
D−(dA)12/BSP
LD−(dA)12/SVP
LD−(dA)12/BSP
0
20
40
60
80
100
120
TIME (min.)

# INTERNATIONAL SEARCH REPORT

International Application No PCT/JP91/01007

**I. CLASSIFICATION OF SUBJECT MATTER** (if several classification symbols apply, indicate all) [6]

According to International Patent Classification (IPC) or to both National Classification and IPC

Int. Cl[5] C07H21/04

**II. FIELDS SEARCHED**

Minimum Documentation Searched [7]

| Classification System | Classification Symbols |
|---|---|
| IPC | C07H19/00-21/04 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| P | Journal of American Chemical Society Vol.112, No.20, pages 7436 to 7438 (1990) | 1-27 |
| X | Biochemical and Biophysical Research Communications, Vol.172, No.2, pages 537 to 543 (1990) | 1-23 |
| X | Nucleosides and Nucleotides, Vol.3, No.5, pages 499 to 512 (1984) | 1 |
| X | Chemical Abstracts Vol.110, No.13, page 738 (1989) 115216t | 1 |
| Y | Journal of Organic Chemistry, Vol.35, No.3, pages 636 to 639 (1970) | 16-23 |
| Y | Chemistry Letters 1983 No.11, pages 1661 to 1664 | 16-23 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| October 4, 1991 (04. 10. 91) | October 21, 1991 (21. 10. 91) |
| **International Searching Authority** | **Signature of Authorized Officer** |
| Japanese Patent Office | |